# EUROPEAN PATENT SPECIFICATION

(11) **EP 2 488 646 B1**
(45) Date of publication and mention of the grant of the patent: **06.12.2017**
(21) Application number: 10779855.5
(22) Date of filing: 14.10.2010
(51) Int. Cl.: C12N 15/113, A61K 31/713, A61P 33/14

(54) **COMPOSITIONS FOR CONTROLLING VARROA MITES IN BEES**
ZUSAMMENSETZUNGEN ZUR STEUERUNG VPN VARROA-MILBEN BEI BIENEN
COMPOSITIONS POUR LUTTER CONTRE LES ACARIENS VARROA CHEZ L'ABEILLE

(30) Priority: 14.10.2009 US 251339 P
(43) Date of publication of application: 22.08.2012
(60) Divisional application: 17188342.4
(73) Proprietor: Yissum Research Development Company of the Hebrew University of Jerusalem Ltd., 91390 Jerusalem (IL); Beeologics Inc., St Louis, MO 63167 (US)
(72) Inventor: SELA, Ilan, St. Louis, Missouri 63167 (US); SHAFIR, Sharoni, St. Louis, Missouri 63167 (US); MAORI, Eyal, St. Louis, Missouri 63167 (US); GARBIAN, Yael, St. Louis, Missouri 63167 (US); BEN-CHANOCH, Eyal, St. Louis, Missouri 63167 (US); YARDEN, Gal, St. Louis, Missouri 63167 (US)
(74) Representative: dompatent von Kreisler Selting Werner - Partnerschaft von Patent- und Rechtsanwälten mbB
(86) International application number: PCT/IL2010/000844
(87) International publication number: WO 2011/045796

(56) References cited:
- WANG RUIWU ET AL: "Molecular characterization of an arachnid sodium channel gene from the varroa mite (Varroa destructor)." INSECT BIOCHEMISTRY AND MOLECULAR BIOLOGY, vol. 33, no. 7, July 2003 (2003-07), pages 733-739, XP002621492 ISSN: 0965-1748
- LIU Z ET AL: "Effect of a fluvalinate-resistance-associated sodium channel mutation from varroa mites on cockroach sodium channel sensitivity to fluvalinate, a pyrethroid insecticide" INSECT BIOCHEMISTRY AND MOLECULAR BIOLOGY, vol. 36, no. 11, 1 November 2006 (2006-11-01), pages 885-889, XP025014535 ELSEVIER SCIENCE LTD, GB ISSN: 0965-1748 DOI: 10.1016/J.IBMB.2006.08.006 [retrieved on 2006-11-01]
- MAORI E ET AL: "IAPV, a bee-affecting virus associated with Colony Collapse Disorder can be silenced by dsRNA ingestion" INSECT MOLECULAR BIOLOGY, vol. 18, no. 1, 1 February 2009 (2009-02-01), pages 55-60, XP002523701 BLACKWELL SCIENTIFIC, OXFORD, GB ISSN: 0962-1075 DOI: 10.1111/J.1365-2583.2009.00847.X
- CAMPBELL EWAN M ET AL: "Gene-knockdown in the honey bee mite Varroa destructor by a non-invasive approach: studies on a glutathione S-transferase." PARASITES & VECTORS, vol. 3, 73, 16 August 2010 (2010-08-16), pages 1-10, XP002621493 ISSN: 1756-3305

### SEQUENCE LISTING

<110> Yissum Research Development Company of the Hebrew
   University of Jerusalem Ltd.
   Beeologics, LLC
   Sela, Ilan
   Shafir, Sharoni
   Maori, Eyal
   Garbian, Yael
   Ben-Chanoch, Eyal
   Yarden, Gal
<120> COMPOSITIONS FOR CONTROLLING VARROA MITES IN BEES
<130> 49981
<150> US 61/251,339
   <151> 2009-10-14
<160> 91
<170> PatentIn version 3.5
<210> 1
   <211> 371
   <212> DNA
   <213> Varroa destructor
<400> 1
   gttagccgtc tgaagcaatg cttgactgta cagggtccga atataaaact tcatacattc      60
   aaaatcacgt atcaggatta tgctaaacat cgcaccataa aaatcttcac taaagttatt     120
   ttacgcttca ggatagtggt ccgttatgag tgttgcggta ttagtgcgtt tacaaatttg     180
   ctaacgatat taacaagctt atttcactcg ttggcaggtt ttctagaacg cgaggtgagg     240
   aaggataacc ttccgatgat gtcattcggc gacaatcctg aggcgcctca gcctcgggag     300
   atgattgatc tagaagcaac ctttgagaaa ctcgaaaacg aactcaatga ggtagttttc     360
   tgtgttgaaa t                                                          371
<210> 2
   <211> 313
   <212> DNA
   <213> Varroa destructor
<400> 2
   ctgtacaggg tccgaatata aaacttcata cattcaaaat cacgtatcag gattatgcta 60
   aacatcgcac cataaaaatc ttcactaaag ttattttacg cttcaggata gtggtccgtt 120
   atgagtgttg cggtattagt gcgtttacag atttgctaac gttattaaca agctaatttc 180
   actcgttggc aggttttcta gaacgcgagg tgaggaagga taaccttccg atgatgtcat 240
   tcggcgacaa tcctgaggcg cctcagcctc gggagatgat tgatctagaa gcaacctttg 300
   agaaactcga aaa 313
<210> 3
   <211> 304
   <212> DNA
   <213> Varroa destructor
<400> 3
   ttcgagtttc tcaaaggttg cttctagatc aatcatctcc cgaggctgag gcgcctcagg 60
   attgtcgccg aatgacatca tcggaaggtt atccttcctc acctcgcgtt ctagaaaacc 120
   tgccaacgag tgaaattagc ttgttaataa cgttagcaaa tctgtaaacg cactaatacc 180
   gcaacactca taacggacca ctatcctgaa gcgtaaaata actttagtga agatttttat 240
   ggtgcgatgt ttagcataat cctgatacgt gattttgaat gtatgaagtt ttatattcgg 300
   accc 304
<210> 4
   <211> 242
   <212> DNA
   <213> Varroa destructor
<400> 4
   caactcatta aaatgaaatc agtattccac atcatgaatc aattgaatat ggacgtcact 60
   cagaagtgtc ttattgccga atgctggatt cctgatcgcg atgtagcaaa ggtacaagct 120
   gccctgcgac gtggaacgga agcggctgga agcagcttcc cgtgtatcat taaccggttg 180
   gaaacggacc aagctccacc gacgttctac agaacgaact cgtttactgc tggctttcaa 240
   aa 242
<210> 5
   <211> 213
   <212> DNA
   <213> Varroa destructor
<400> 5
   caattcgtga ttcaattgaa tatggacgtc actcagaagt gtcttattgc cgaatgctgg 60
   attcctgatc gcgatgtagc aaaggtacaa gctgccctgc gacgtggaac ggaagcggct 120
   ggaagcagct tcccgtgtat cattaaccgg ttggaaacgg acaaagctcc accgacgttc 180
   tacagaacga actcgtttac tgctggcttt caa 213
<210> 6
   <211> 201
   <212> DNA
   <213> Varroa destructor
<400> 6
   caattgaata tggacgtcac tcagaagtgt cttattgccg aatgctggat tcctgatcgc 60
   gatgtagcaa aggtacaagc tgccctgcga cgtggaacgg aagcggctgg aagcagcttc 120
   ccgtgtatca ttaaccggtt ggaaacggac aaagctccac cgacgttcta cagaacgaac 180
   tcgtttattg ctggctttca a 201
<210> 7
   <211> 307
   <212> DNA
   <213> Varroa destructor
<400> 7
   aatcacaatt ttctacatca tcttcttcat ctgcttggcg gcattctgga cggttatgct 60
   ggtcatcttc tatcagacac tcgatgcctt ccagccaaag tggaccctgg acgctagtct 120
   cattggcact gtaccgggat taggcttcag gccacgccca ccgctgtcta acatcgactc 180
   aacactcatc tatttcaagg tatctaagcc gttagtgtat atgttatatt atagcgctct 240
   ttgttatgtg gaaagacgcc agggcgcgta tctatatggt ggttttcata ccaaccgtgg 300
   gaaccca 307
<210> 8
   <211> 290
   <212> DNA
   <213> Varroa destructor
<400> 8
   ggttcccacg gttggtatga aaaccaccat atagatacgc gccctggcgt ctttccacat 60
   aacaaagagc gctataatat aacatataca ctaacggctt agataccttg aaatagatga 120
   gtgttgagtc gatgttagac agcggtgggc gtggcctgaa gcctaatccc ggtacagtgc 180
   caatgagact agcgtccagg gtccactttg gctggaaggc atcgagtgtc tgatagaaga 240
   tgaccagcat aaccgtccag aatgccgcca agcagatgaa gaagatgatg 290
<210> 9
   <211> 290
   <212> DNA
   <213> Varroa destructor
<400> 9
   catcatcttc ttcatctgct tggcggcatt ctggacggtt atgctggtca tcttctatca     60
   gacactcgat gccttccagc caaagtggac cctggacgct agtctcattg gcactgtacc    120
   gggattaggc ttcaggccac gcccaccgct gtctaacatc gactcaacac tcatctattt    180
   caaggtatct aagccgttag tgtatatgtt atattatagc gctctttgtt atgtggaaag    240
   acgccagggc gcgtatctat atggtggttt tcataccaac cgtgggaacc               290
<210> 10
   <211> 357
   <212> DNA
   <213> Varroa destructor
<400> 10
   gaggtgacat ccgtgttcgc cgtgtacggc atcaaagtgg atccaagaca tctaagtctg     60
   gtaggggact acatgacttt cgacggagct taccgcgcct tcaacagaat ccacatggca    120
   aacaatgcat cgccactcca gcagatgagc tttgaaacga cgtgcacatt tatgaaaaac    180
   gctgctttat ttggtacgaa atcccctaag acagatacga agacaatctt tgccatgcta    240
   atagtgtttc tgtttttagt gcctggtacg atcattaatt acggcgttga aagtaactcc    300
   aaacagcgac cctatatgtc ttcatacaag agacttagtt ctaggaaagc aaataca       357
<210> 11
   <211> 325
   <212> DNA
   <213> Varroa destructor
<400> 11
   tatgaagaca tatagggtcg ctgtttggag ttactttcaa cgccgtaatt aatgatcgta     60
   ccaggcacta aaaacagaaa cactattagc atggcaaaga ttgtcttcgt atctgtctta    120
   ggggatttcg taccaaataa agcagcgttt ttcataaatg tgcacgtcgt ttcaaagctc    180
   atctgctgga gtggcgatgc attgtttgcc atgtggattc tgttgaaggc gcggtaagct    240
   ccgtcgaaag tcatgtagtc ccctaccaga cttagatgtc ttggatccac tttgatgccg    300
   tacacggcga acacggatgt cacct                                          325
<210> 12
   <211> 324
   <212> DNA
   <213> Varroa destructor
<400> 12
   atgaagacat atagggtcgc tgtttggagt tactttcaac gccgtaatta atgatcgtac     60
   caggcactaa aaacagaaac actattagca tggcaaagat tgtcttcgta tctgtcttag    120
   gggatttcgt accaaataaa gcagcgtttt tcataaatgt gcacgtcgtt tcaaagctca    180
   tctgctggag tggcgatgca ttgtttgcca tgtggattct gttgaaggcg cggtaagctc 240
   cgtcgaaagt catgtagtcc cctaccagac ttagatgtct tggatccact ttgatgccgt 300
   acacggcgaa cacggatgtc acct 324
<210> 13
   <211> 406
   <212> DNA
   <213> Varroa destructor
<400> 13
   cctgcgcatc catcagatga ttggaacgga ggaaaatgtc caagtagcat tcgtgggctc 60
   gattgtcgag tgtcacaagc tcaaggtgtt tactcaggaa gaagcactga gattccttgc 120
   ggcaaagatg aagcagcgga tgtttggacc acagaaagcg gaagacccct tgacaaggca 180
   tgggaagccg tactttcatc cgtagtcaac catattcccg ttcaatcgcc tgactacaat 240
   atgactgtcc gggcacacta tcttgcacta atggtgcgtc gcatcattca ggcgcgttat 300
   gatcgccgct tcattgacga tcgcgactat tacggcaaca aacgaattga gcttccgggt 360
   tcgatgatat cgctgctgtt tgaagacctg ttaaaaaagg ttaatg 406
<210> 14
   <211> 283
   <212> DNA
   <213> Varroa destructor
<400> 14
   tcagatttca gatgattgga acggaggaaa atgtccaagt agcattcgtg ggctcgattg 60
   tcgagtgtca caagctcaag gtgtttactc aggaagaagc actgagattc cttgcggcaa 120
   agatgaagca gcggatgttt ggaccacaga aagcggaaga cccccttgac aaggcatggg 180
   aagccgtact ttcatccgta gtcaaccata ttcccgttca atcgcctgac tacaatatga 240
   ccgtccgggc acactatctt gcactaatgg tgcgtcacat cat 283
<210> 15
   <211> 381
   <212> DNA
   <213> Varroa destructor
<400> 15
   tcagatgatt ggaacggagg aaaatgtcca agtagcattc gtgggctcga ttgtcgagtg 60
   tcacaagctc aaggtgttta ctcaggaaga agcactgaga ttccttgcgg caaagatgaa 120
   gcagcggatg tttggaccac agaaagcgga agaccccctt gacaaggcat gggaagccgt 180
   actttcatcc gtagtcaacc atattcccgt tcaatcgcct gactacaata tgaccgtccg 240
   ggcacactat cttgcactaa tggtgcgtca catcattcag gcgcgttatg atcgccgctt 300
   cattgacgat cgcgactatt acggcaacaa acgaattgag cttccgggtt cgatgatatc 360
   gctgctgttt gaagacctgt t 381
<210> 16
   <211> 446
   <212> DNA
   <213> Varroa destructor
<400> 16
   aatcaattcg tctgcagatc tcaccgattt tctgatatcg ctgggagtcc aggatattcg 60
   actactatgc ggagctgaat tcagcaaaac acacgtctac tatgtattcc acaacggtgt 120
   tattaaaggc gtcgttgagg atcatcgcag gcttatcaac gagattcggc aatttcgtcg 180
   gaagggatac ttgtcgcctt acttatcagt ttatccaaat catctacatc gctgtgtgta 240
   tattgtaact gacggtggtc gtttctgcag gccgtttatc attgttgagg atggtcagcc 300
   aaaagttacg cagaaacatt tggacgacct caaagccaat atatataact tccaagactt 360
   cctggacatg ggctttgtag agtttctcga tgtaaatgag gaaaacgacg cgcttatcgc 420
   catttatgaa aaagatatca caatca 446
<210> 17
   <211> 428
   <212> DNA
   <213> Varroa destructor
<400> 17
   ttcataaatg gcgataagcg cgtcgttttc ctcatttaca tcgagaaact ctacaaagcc 60
   catgtccagg aagtcttgga agttatatat attggctttg aggtcgtcca aatgtttctg 120
   cgtaactttt ggctgaccat cctcaacaat gataaacggc ctgcagaaac gaccaccgtc 180
   agttacaata tacacacagc gatgtagatg atttggataa actgataagt aaggcgacaa 240
   ctatcccttc cgacgaaatt gccgaatctc gttgataagc ctgcgatgat cctcaacgac 300
   gcctttaata acaccgttgt ggaatacata gtagacgtgt gttttgctga attcagctcc 360
   gcatagtagt cgaatatcct ggactcccag cgatatcaga aaatcggtga gatctgcaga 420
   cgaattga 428
<210> 18
   <211> 426
   <212> DNA
   <213> Varroa destructor
<400> 18
   tcaattcgtc tgcagatctc accgattttc tgatatcgct gggaatccag gatattcgac     60
   tactatgcgg agctgaattc agcaaaacac acgtctacta tgtattccac aacggtgtta    120
   ttaaaggcgt cgttgaggat catcgcaggc ttatcaacga gattcggcaa tttcgtcgga    180
   agggatactt gtcgccttac ttatcagttt atccaaatca tctacatcgc tgtgtgtata    240
   ttgtaactga cggtggtcgt ttctgcaggc cgtttatcat tgttgaggat ggtcagccaa    300
   aagttacgca gaaacatttg gacgacctca aagccaatat atataacttc caagacttcc    360
   tggacatggg ctttgtagag tttctcgatg taaatgagga aaacgacgcg cttatcgcca    420
   tttatg                                                               426
<210> 19
   <211> 378
   <212> DNA
   <213> Varroa destructor
<400> 19
   gttttgaaca aaatgagtgt tgagcgcgga tttaaggccg gtgtagtata taaaacagaa     60
   acgatcaatt tgcgtaagtt atctggggat gtgggagtcc agacatcgtg cgtttttggt    120
   cgaaaggcag gagattctga gttacagaaa tttgtagatg ttgatggcct gccatacatc    180
   ggcagcaggg tagtacaggg agatccggta tgtgcatata taaatttgac cacgggacaa    240
   ctgaagactg taaggtatta ctcgaccgag ccagcaatcg tgcatgaagt gaaaattctt    300
   ggtaatgatt ccggtacaga caccctccaa caaatccagt tgacgtatct tattgatcga    360
   acgccaaatg atcggaga                                                  378
<210> 20
   <211> 367
   <212> DNA
   <213> Varroa destructor
<400> 20
   aatgagtgtt gagcgcggat ttaaggccgg tgtagtatat aaaacagaaa cgatcaattt     60
   gcgtaagtta tctggggatg tgggagtcca gacatcgtgc gtttttggtc gaaaggcagg    120
   agattctgag ttacagaaat ttgtagatgt tgatggcctg ccatacatcg gcagcagggt    180
   agtacaggga gatccggtat gtgcatatat aaatttgacc acgggacaac tgaagactgt    240
   aaggtattac tcgaccgagc cagcaatcgt gcatgaagtg aaaattcttg gtaatgattc    300
   cggtacagac accctccaac aaatccagtt gacgtatctt gttgatcgaa cgccaaatga    360
   tcggaga                                                              367
<210> 21
   <211> 366
   <212> DNA
   <213> Varroa destructor
<400> 21
   aatgagtgtt gagcgcggat ttaaggccgg tgtagtatat aaaacagaaa cgatcaattt 60
   gcgtaagtta tctggggatg tgggagtcca gacatcgtgc gtttttggtc gaaaggcagg 120
   agattctgag ttacagaaat ttgtagatgt tgatggcctg ccatacatcg gcagcagggt 180
   agtacaggga gatccggtat gtgcatatat aaatttgacc acgggacaac tgaagactgt 240
   aaggtattac tcgaccgagc cagcaatcgt gcatgaagtg aaaattcttg gtaatgattc 300
   cggtacagac accctccaac aaatccagct gacgtatctt gttgatcgaa cgccaaatga 360
   tcggag 366
<210> 22
   <211> 268
   <212> DNA
   <213> Varroa destructor
<400> 22
   aatggtttct gctacctgtg aggatagtat gcgggatgct tgtattcgtt ttcttgcctc 60
   gaaagtcaat ctcaaagcgc ttgacagtga gacagagctt atgctcattg aagaggccgg 120
   caaagtggca gccctcgtcg gtggagagga gtttgtgctg ctggttaagc tcctcaattc 180
   attaaaggta gattgtacat tttggcgtct tctcgaacaa gttagaatct atttagcaaa 240
   gtgccaatgt atcagcttcc aatacgca 268
<210> 23
   <211> 263
   <212> DNA
   <213> Varroa destructor
<400> 23
   attggaagct gatacattgg cactttgcta aatagattct aacttgttcg agaagacgcc 60
   aaaatgtaca atctaccttt aatgaattga ggagcttaac cagcagcaca aactcctctc 120
   caccgacgag ggctgccact ttgccggcct cttcaatgag cataagctct gtctctctgt 180
   caagcgcttt gagattgact ttcgaggcaa gaaaacgaat acaagcatcc cgcatactat 240
   cctcacaggt agcagaaacc att 263
<210> 24
   <211> 263
   <212> DNA
   <213> Varroa destructor
<400> 24
   attggaagct gatacattgg cactttgcta aatagattct aacttgttcg agaagacgcc 60
   aaaatgtaca atctaccttt aatgaattga ggagcttaac cagcagcaca aactcctctc 120
   caccgacgag ggctgccact ttgccggcct cttcaatgag cataagctct gtctctctgt 180
   caagcgcttt gagattgact ttcgaggcaa gaaaacgaat acaagcatcc cgcatactat 240
   cctcacaggt agcagaaacc att 263
<210> 25
   <211> 279
   <212> DNA
   <213> Varroa destructor
<400> 25
   gatcttgttg aagccggctt cttcttcctt ggcatgcacg attacacgaa atgcttccat 60
   tgcgacggcg gtctgtgtaa ttgggagaca ggtgacgacc cctgggtaga gcatgcccgc 120
   tggttccctg aatgtcaatt cgttcagcta agcaagggcg gagcattcat cgctgagtgc 180
   caacaacgtc acgaaaaact agttaatggc gcggtagccc aggcagaact tcaggctttt 240
   agtgaagtag aaccgggagg aacaggcagt gactcaaat 279
<210> 26
   <211> 263
   <212> DNA
   <213> Varroa destructor
<400> 26
   agtcactgcc tgttcctccc ggttctactt cactaaaagc ctgaagttct gcctgggcta 60
   ccgcgccatt aactagtttt tcgtgacgtt gttggcactc agcgatgaat gctccgccct 120
   tgcttagctg aacgaattga cattcaggga accagcgggc atgctctacc caggggtcgt 180
   cacctgtctc ccaattacac agaccgccgt cgcaatggaa gcatttcgtg taatcgtgca 240
   tgccaaggaa gaagaagccg gct 263
<210> 27
   <211> 299
   <212> DNA
   <213> Varroa destructor
<400> 27
   ttccgcttca tttgagaact gagcttgaag aaataatgca gtcgcccgtc gtcaagttct 60
   acctcgagaa aggtgtaccg aaacaagtga ttcgaatgac cgtaaaaaat atatgcttga 120
   caacgagcgc ggtttccgtg atcttgacga aattacacac gtactcggac aggtgctcag 180
   cttcggcaac aagaagactg cgcctgccaa tgaaaaaggt aggtggatac cggatatttg 240
   tcgggaattc aatgcagctg aacccgatga ggttgattca gaattggcat acaatagaa 299
<210> 28
   <211> 284
   <212> DNA
   <213> Varroa destructor
<400> 28
   ttccgcttca tttgagaact gagcttgaag aaataatgca gtcgcccgtc gtcaagttct      60
   acctcgagaa aggtgtaccg aaacaagtga ttcgaatgac cgtaaaaaaa tatatgcttg     120
   acaacgagcg cggtttccgt gatcttgacg aaattacaca cgtactcgga caggtgctca     180
   gcttcggcaa caagaagact gcgcctgcca atgaaaaagg taggtggata ccggatattt     240
   gtcgggaatt caatgcagct gaacccgatg aggttgattc agaa                      284
<210> 29
   <211> 283
   <212> DNA
   <213> Varroa destructor
<400> 29
   ttccgcttca tttgagaact gagcttgaag aaataatgca gtcgcccgtc gtcaagttct      60
   acctcgagaa aggtgtaccg aaacaagtga ttcgaatgac cgtgaaaaaa tatatgcttg     120
   acaacgagcg cggtttccgt gatcttgacg aaattacaca cgtactcgga caggtgctca     180
   gcttcggcaa caagaagact gcgcctgcca atgaaaaagg taggtggata ccggatattt     240
   gtcgggaatt caatgcagct gaacccgatg aggttgattc aga                       283
<210> 30
   <211> 279
   <212> DNA
   <213> Varroa destructor
<400> 30
   tggctaatta atagtaggcc gaagaacttt ttgagtggcc tcgatatgtc cgacgttgtg      60
   gcttcgtggg aggttccttt ggttggccaa gcttaccgag tcgaattcga acacggaagt     120
   gcaacgggta aacgtgttgt gtacgttaat ggactcgagg tgttacgaaa acactggctt     180
   tttaagcttg ttggcgagga aagctttgac atattgggac ataagtgcat catttctatc     240
   aaagccgtag gaggcttcag gttggtagca aactccagt                            279
<210> 31
   <211> 277
   <212> DNA
   <213> Varroa destructor
<400> 31
   tggctaatta atagtaggcc gaagaacttt ttgagtggcc tcgatatgtc cgacgttgtg 60
   gcttcgtggg aggttccttt ggttggccaa gcttaccgag tcgaattcga acacggaagt 120
   gcaacgggta aacgtgttgt gtacgttaat ggactcgagg tgttacgaaa acactggctt 180
   tttaagcttg ttggcgagga aagctttgac atattgggac ataagtgcat catttctatc 240
   aaagccgtag gaggcttcag gttggtagca aactcca 277
<210> 32
   <211> 277
   <212> DNA
   <213> Varroa destructor
<400> 32
   tggagtttgc taccaacctg aagcctccta cggctttgat agaaatgatg cacttatgtc 60
   ccaatatgtc aaagctttcc tcgccaacaa gcttaaaaag ccagtgtttt cgtaacacct 120
   cgagtccatt aacgtacaca acacgtttac ccgttgcact tccgtgttcg aattcgactc 180
   ggtaagcttg gccaaccaaa ggaacctccc acgaagccac aacgtcggac atatcgaggc 240
   cactcaaaaa gttcttcggc ctactattaa ttagcca 277
<210> 33
   <211> 283
   <212> DNA
   <213> Varroa destructor
<400> 33
   ggtcttgaca acacatgcta ccctcgaaca cgccgactgc gtcttcatga tggacaatga 60
   ggccatctat cagatctgcc gtcggaacct tggagtcgag cgaccggcgt accagaatct 120
   caaccgtctg atcagtcagg ccgtttcggc gattaccgct tctctacgtt tctccggagc 180
   gctgaatgtt gatcttaacg agttccaaac taatttagtt ccatacccgc gaatccattt 240
   tcccctcgtc acttacgctc cgatcatttc tgctgagaag gct 283
<210> 34
   <211> 279
   <212> DNA
   <213> Varroa destructor
<400> 34
   ggtcttgaca acacatgcta ccctcgaaca cgccgactgc gtcttcatga tggacaatga 60
   ggccatctat cagatctgcc gtcggaacct tggagtcgag cgaccggcgt accagaatct 120
   caaccgtctg atcagtcagg ccgtttcggc gattaccgct tctctacgtt tctccggagc 180
   gctgaatgtt gatcttaacg agttccaaac taatttagtt ccatacccgc gaatccattt 240
   tccccccgtc acttacgctc cgatcatttc tgctgagaa 279
<210> 35
   <211> 277
   <212> DNA
   <213> Varroa destructor
<400> 35
   ctcagcagaa atgatcggag cgtaagtgac ggggggaaaa tggattcgcg ggtatggaac      60
   taaattagtt tggaactcgt taagatcaac attcagcgct ccggagaaac gtagagaagc     120
   ggtaatcgcc gaaacggcct gactgatcag acggttgaga ttctggtacg ccggtcgctc     180
   gactccaagg ttccgacggc agatctgata gatggcctca ttgtccatca tgaagacgca     240
   gtcggcgtgt tcgagggtag catgtgttgt caagacc                              277
<210> 36
   <211> 332
   <212> DNA
   <213> Varroa destructor
<400> 36
   caacgctgtg cttcacgtag actccacgtt cgaaaatgtc gactgcacgt ttatggttga      60
   taatcaaaca ctcttcaagc tttgtcgaga ccggctaaag attaggagtc catcttatga     120
   caacgcaaat gctgtcattt cccagggttt ttcgtcaatc atgaattcgg tggggctgga     180
   tggatccttg aatgtggacc tcagcgagtt ccaaacaaat ctcgtccctt ttggaagatt     240
   acattttacg atgatgagct acagtccatt cgttacatcc ggacaccgcg atctaagccg     300
   tgagacgtcc gtcgtggaga ttactcgtga cc                                   332
<210> 37
   <211> 330
   <212> DNA
   <213> Varroa destructor
<400> 37
   caacgctgtg cttcacgtag actccacgtt cgaaaatgtc gactgcacgt ttatggttga      60
   taatcaaaca ctcctcaagc tttgtcgaga ccggctaaag gttaggagtc catcttatga     120
   caacgcaaat gctgtcattt cccagggttt ttcgtcaatc atgaattcgg tggggctgga     180
   tggatccttg aatgtggacc tcagcgagtt ccaaacaagt ctcgtccctt ttggaagatt     240
   acattttacg atgatgagct acagtccatt cgttacatcc ggacaccgcg atctaagccg     300
   tgagacgtcc gtcgtggaga ttactcgtga                                      330
<210> 38
   <211> 329
   <212> DNA
   <213> Varroa destructor
<400> 38
   aacgctgtgc ttcacgtaga ctccacgttc gaaaatgtcg actgcacgtt tatggttgat 60
   aatcaaacac tcctcaagct ttgtcgagac cggctaaagg ttaggagtcc atcttatgac 120
   aacgcaaatg ctgtcatttc ccagggtttt tcgtgaatca tgaattcggt ggggctggat 180
   ggatccttga atgtggacct cagcgagttc caaacaagtc tcgtcccttt tggaagatta 240
   cattttacga tgatgagcta cagtccattc gttacatccg gacaccgcga tctaagccgt 300
   gagacgtccg tcgtggagat tactcgtga 329
<210> 39
   <211> 415
   <212> DNA
   <213> Varroa destructor
<400> 39
   tatggagaac atcgcacagg acttcggtaa aaagtgccga ttgggcttcg ccatctaccc 60
   ggctccgcag gtttccactg ccgttgtcga accatacaac tcggttttga cgacacatgc 120
   caccctcgaa cacgctgact gcgtattcat gatggataat gaggcgatct atcagatctg 180
   tcgtcggaat cttggagttg aacgaccggc gtatcaaaat ctcaatcgac tgattagcca 240
   ggccgtttcg gcgataaccg cttctctacg tttttccgga gcgttgaatg ttgacctcaa 300
   cgaatttcag acgaatctcg tcccctaccc gcgaatccat ttcccgctcg tcacttatgc 360
   tccgattatt tcggctgaga aggctcatca cgagcaacat aacgtactgg aaatc 415
<210> 40
   <211> 412
   <212> DNA
   <213> Varroa destructor
<400> 40
   atggagaaca tcgcacagga cttcggtaaa aagtgccgat tgggcttcgc catctacccg 60
   gctccgcagg tttccactgc cgttgtcgaa ccatacaact cggttttgac gacacatgcc 120
   accctcgaac acgctgactg cgtattcatg atggataatg aggcgatcta tcagatctgt 180
   cgtcggaatc ttggagttga acgaccggcg tatcaaaatc tcaatcgact gattagccag 240
   gccgtttcgg cgataaccgc ttctctacgt ttttccggag cgttgaatgt tgacctcaac 300
   gaatttcaga cgaatctcgt cccctacccg cgaatccatt tcccgctcgt cacttatgct 360
   ccgattattt cggctgagaa ggctcatcac gagcaacata acgtactgga aa 412
<210> 41
   <211> 411
   <212> DNA
   <213> Varroa destructor
<400> 41
   atggagaaca tcgcacagga cttcggtaaa aagtgccgat tgggcttcgc catctacccg    60
   gctccgcagg tttccactgc cgttgtcgaa ccatacaact cggttttgac gacacatgcc   120
   accctcgaac acgctgactg cgtattcatg atggataatg aggcgatcta tcagatctgt   180
   cgtcggaatc ttggagttga acgaccggcg tatcaaaatc tcaatcgact gattagccag   240
   gccgtttcgg cgataaccgc ttctctacgt ttttccggag cgttgaatgt tgacctcaac   300
   gaatttcaga cgaatctcgt cccctacccg cgaatccatt tcccgctcgt cacttatgct   360
   ccgattattt cggctgagaa ggctcatcac gagcaacata acgtactgga a             411
<210> 42
   <211> 266
   <212> DNA
   <213> Varroa destructor
<400> 42
   attttattca attaaagtat ttaccaattg gaataaagat aggattaatg ataatttttt    60
   taagtttaag tggaatacct ccctttatag gatttatttc taagataact gttttgttga   120
   tgtattttga gaatcaaaaa ataatttttt taattatatt attagtatct gtaataagaa   180
   tatatattta tataaattat tttatgaaga gtttattttt tataagatta ggttataata   240
   aaaataaaaa tataggaata agaaga                                         266
<210> 43
   <211> 99
   <212> DNA
   <213> Varroa destructor
<400> 43
   atacctcaaa tgtatccttc atattgagta ttaattcata tagtatttat attgaattat    60
   tatataataa taatttatta ttattttata tttaagtaa                            99
<210> 44
   <211> 501
   <212> DNA
   <213> Varroa destructor
<400> 44
   ggccaatccc gattccggcg acacaaagaa attacaagaa gctatagacc gttttcatcg      60
   agccggaaga tggattaaga aaaaatttcg agatctattc atgctatgtt cgggtaaaca     120
   gcgcaaccag atctcggatc aaacctacgc cgaagacctg gacctcgaca caggggtcat     180
   tattatggat ggacaggtta ttaagaagga tagccccacg cccgaactca tcgatgggtt     240
   ggatgttggt tttcaagctg ataagcaaca ggcgcaggtg attgtaatgc aaaagcttaa     300
   aaacaattcc cgacctatca ttggcgactc aaaggaattt agcaacaaag ttcatccagg     360
   ccccgacttt tgcctggtaa agccgaacga caacggcgaa ggcctcgtgc aagacaccga     420
   gcttggggcc tccacgccgc tcagctcgcc ttcctgtata gttgaacagc ctctgtctca     480
   cgacagtgtg ggcctgccac c                                               501
<210> 45
   <211> 21
   <212> DNA
   <213> Artificial sequence
<220>
   <223> Exemplary siRNA targeting sequence
<400> 45
   attttattca attaaagtat t 21
<210> 46
   <211> 21
   <212> DNA
   <213> Artificial sequence
<220>
   <223> Exemplary siRNA targeting sequence
<400> 46
   atacctcaaa tgtatccttc a 21
<210> 47
   <211> 21
   <212> DNA
   <213> Artificial sequence
<220>
   <223> Exemplary siRNA targeting sequence
<400> 47
   ggccaatccc gattccggcg a 21
<210> 48
   <211> 24
   <212> DNA
   <213> Artificial sequence
<220>
   <223> T7 promoters
<400> 48
   ctaatacgac tcactatagg gcga 24
<210> 49
   <211> 24
   <212> DNA
   <213> Artificial sequence
<220>
   <223> T7 promoters (reverse complement orientation)
<400> 49
   tcgccctata gtgagtcgta ttag 24
<210> 50
   <211> 419
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (395)..(419)
   <223> Promoter
<400> 50
   ctaatacgac tcactatagg gcgagttagc cgtctgaagc aatgcttgac tgtacagggt      60
   ccgaatataa aacttcatac attcaaaatc acgtatcagg attatgctaa acatcgcacc     120
   ataaaaatct tcactaaagt tattttacgc ttcaggatag tggtccgtta tgagtgttgc     180
   ggtattagtg cgtttacaaa tttgctaacg atattaacaa gcttatttca ctcgttggca     240
   ggttttctag aacgcgaggt gaggaaggat aaccttccga tgatgtcatt cggcgacaat     300
   cctgaggcgc ctcagcctcg ggagatgatt gatctagaag caacctttga gaaactcgaa     360
   aacgaactca atgaggtagt tttctgtgtt gaaattcgcc ctatagtgag tcgtattag      419
<210> 51
   <211> 361
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (337)..(361)
   <223> Promoter
<400> 51
   ctaatacgac tcactatagg gcgactgtac agggtccgaa tataaaactt catacattca      60
   aaatcacgta tcaggattat gctaaacatc gcaccataaa aatcttcact aaagttattt     120
   tacgcttcag gatagtggtc cgttatgagt gttgcggtat tagtgcgttt acagatttgc     180
   taacgttatt aacaagctaa tttcactcgt tggcaggttt tctagaacgc gaggtgagga     240
   aggataacct tccgatgatg tcattcggcg acaatcctga ggcgcctcag cctcgggaga     300
   tgattgatct agaagcaacc tttgagaaac tcgaaaatcg ccctatagtg agtcgtatta     360
   g                                                                     361
<210> 52
   <211> 359
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (335)..(359)
   <223> Promoter
<400> 52
   ctaatacgac tcactatagg gcgattcgag tttctcaaag gttgcttcta gatcaatcat      60
   ctcccgaggc tgaggcgcct caggattgtc gccgaatgac atcatcggaa ggttatcctt     120
   cctcacctcg cgttctagaa aacctgccaa cgagtgaaat tagcttgtta ataacgttag     180
   caaatctgta aacgcactaa taccgcaaca ctcataacgg accactatcc tgaagcgtaa     240
   aataacttta gtgaagattt ttatggtgcg atgtttagca taatcctgat acgtgatttt     300
   gaatgtatga agttttatat tcggaccctg tacagtcgcc ctatagtgag tcgtattag      359
<210> 53
   <211> 290
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (266)..(290)
   <223> Promoter
<400> 53
   ctaatacgac tcactatagg gcgacaactc attaaaatga aatcagtatt ccacatcatg      60
   aatcaattga atatggacgt cactcagaag tgtcttattg ccgaatgctg gattcctgat     120
   cgcgatgtag caaaggtaca agctgccctg cgacgtggaa cggaagcggc tggaagcagc     180
   ttcccgtgta tcattaaccg gttggaaacg gaccaagctc caccgacgtt ctacagaacg     240
   aactcgttta ctgctggctt tcaaaatcgc cctatagtga gtcgtattag                290
<210> 54
   <211> 261
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (237)..(261)
   <223> Promoter
<400> 54
   ctaatacgac tcactatagg gcgacaattc gtgattcaat tgaatatgga cgtcactcag      60
   aagtgtctta ttgccgaatg ctggattcct gatcgcgatg tagcaaaggt acaagctgcc     120
   ctgcgacgtg gaacggaagc ggctggaagc agcttcccgt gtatcattaa ccggttggaa     180
   acggacaaag ctccaccgac gttctacaga acgaactcgt ttactgctgg ctttcaatcg     240
   ccctatagtg agtcgtatta g                                               261
<210> 55
   <211> 249
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (225)..(249)
   <223> Promoter
<400> 55
   ctaatacgac tcactatagg gcgacaattg aatatggacg tcactcagaa gtgtcttatt       60
   gccgaatgct ggattcctga tcgcgatgta gcaaaggtac aagctgccct gcgacgtgga      120
   acggaagcgg ctggaagcag cttcccgtgt atcattaacc ggttggaaac ggacaaagct      180
   ccaccgacgt tctacagaac gaactcgttt attgctggct ttcaatcgcc ctatagtgag      240
   tcgtattag                                                              249
<210> 56
   <211> 355
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (331)..(355)
   <223> Promoter
<400> 56
   ctaatacgac tcactatagg gcgaaatcac aattttctac atcatcttct tcatctgctt       60
   ggcggcattc tggacggtta tgctggtcat cttctatcag acactcgatg ccttccagcc      120
   aaagtggacc ctggacgcta gtctcattgg cactgtaccg ggattaggct tcaggccacg      180
   cccaccgctg tctaacatcg actcaacact catctatttc aaggtatcta agccgttagt      240
   gtatatgtta tattatagcg ctctttgtta tgtggaaaga cgccagggcg cgtatctata      300
   tggtggtttt cataccaacc gtgggaaccc actaatacga ctcactatag ggcga           355
<210> 57
   <211> 338
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (314)..(338)
   <223> Promoter
<400> 57
   ctaatacgac tcactatagg gcgaggttcc cacggttggt atgaaaacca ccatatagat     60
   acgcgccctg gcgtctttcc acataacaaa gagcgctata atataacata tacactaacg    120
   gcttagatac cttgaaatag atgagtgttg agtcgatgtt agacagcggt gggcgtggcc    180
   tgaagcctaa tcccggtaca gtgccaatga gactagcgtc cagggtccac tttggctgga    240
   aggcatcgag tgtctgatag aagatgacca gcataaccgt ccagaatgcc gccaagcaga    300
   tgaagaagat gatgtcgccc tatagtgagt cgtattag                            338
<210> 58
   <211> 338
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (314)..(338)
   <223> Promoter
<400> 58
   ctaatacgac tcactatagg gcgacatcat cttcttcatc tgcttggcgg cattctggac     60
   ggttatgctg gtcatcttct atcagacact cgatgccttc cagccaaagt ggaccctgga    120
   cgctagtctc attggcactg taccgggatt aggcttcagg ccacgcccac cgctgtctaa    180
   catcgactca acactcatct atttcaaggt atctaagccg ttagtgtata tgttatatta    240
   tagcgctctt tgttatgtgg aaagacgcca gggcgcgtat ctatatggtg gttttcatac    300
   caaccgtggg aacctcgccc tatagtgagt cgtattag                            338
<210> 59
   <211> 405
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (381)..(405)
   <223> Promoter
<400> 59
   ctaatacgac tcactatagg gcgagaggtg acatccgtgt tcgccgtgta cggcatcaaa 60
   gtggatccaa gacatctaag tctggtaggg gactacatga ctttcgacgg agcttaccgc 120
   gccttcaaca gaatccacat ggcaaacaat gcatcgccac tccagcagat gagctttgaa 180
   acgacgtgca catttatgaa aaacgctgct ttatttggta cgaaatcccc taagacagat 240
   acgaagacaa tctttgccat gctaatagtg tttctgtttt tagtgcctgg tacgatcatt 300
   aattacggcg ttgaaagtaa ctccaaacag cgaccctata tgtcttcata caagagactt 360
   agttctagga aagcaaatac atcgccctat agtgagtcgt attag 405
<210> 60
   <211> 373
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (349)..(373)
   <223> Promoter
<400> 60
   ctaatacgac tcactatagg gcgatatgaa gacatatagg gtcgctgttt ggagttactt 60
   tcaacgccgt aattaatgat cgtaccaggc actaaaaaca gaaacactat tagcatggca 120
   aagattgtct tcgtatctgt cttaggggat ttcgtaccaa ataaagcagc gtttttcata 180
   aatgtgcacg tcgtttcaaa gctcatctgc tggagtggcg atgcattgtt tgccatgtgg 240
   attctgttga aggcgcggta agctccgtcg aaagtcatgt agtcccctac cagacttaga 300
   tgtcttggat ccactttgat gccgtacacg gcgaacacgg atgtcacctt cgccctatag 360
   tgagtcgtat tag 373
<210> 61
   <211> 372
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (348)..(372)
   <223> Promoter
<400> 61
   ctaatacgac tcactatagg gcgaatgaag acatataggg tcgctgtttg gagttacttt      60
   caacgccgta attaatgatc gtaccaggca ctaaaaacag aaacactatt agcatggcaa     120
   agattgtctt cgtatctgtc ttaggggatt tcgtaccaaa taaagcagcg tttttcataa     180
   atgtgcacgt cgtttcaaag ctcatctgct ggagtggcga tgcattgttt gccatgtgga     240
   ttctgttgaa ggcgcggtaa gctccgtcga aagtcatgta gtcccctacc agacttagat     300
   gtcttggatc cactttgatg ccgtacacgg cgaacacgga tgtcaccttc gccctatagt     360
   gagtcgtatt ag                                                         372
<210> 62
   <211> 453
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (429)..(453)
   <223> Promoter
<400> 62
   ctaatacgac tcactatagg gcgacctgcg catccatcag atgattggaa cggaggaaaa 60
   tgtccaagta gcattcgtgg gctcgattgt cgagtgtcac aagctcaagg tgtttactca 120
   ggaagaagca ctgagattcc ttgcggcaaa gatgaagcag cggatgtttg gaccacagaa 180
   agcggaagac cccttgacaa ggcatgggaa gccgtacttt catccgtagt caaccatatt 240
   cccgttcaat cgcctgacta caatatgact gtccgggcac actatcttgc actaatggtg 300
   cgtcgcatca ttcaggcgcg ttatgatcgc cgcttcattg acgatcgcga ctattacggc 360
   aacaaacgaa ttgagcttcc gggttcgatg atatcgctgc tgtttgaaga cctgttaaaa 420
   aaggttaatt cgccctatag tgagtcgtat tag 453
<210> 63
   <211> 331
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (307)..(331)
   <223> Promoter
<400> 63
   ctaatacgac tcactatagg gcgatcagat ttcagatgat tggaacggag gaaaatgtcc 60
   aagtagcatt cgtgggctcg attgtcgagt gtcacaagct caaggtgttt actcaggaag 120
   aagcactgag attccttgcg gcaaagatga agcagcggat gtttggacca cagaaagcgg 180
   aagaccccct tgacaaggca tgggaagccg tactttcatc cgtagtcaac catattcccg 240
   ttcaatcgcc tgactacaat atgaccgtcc gggcacacta tcttgcacta atggtgcgtc 300
   acatcattcg ccctatagtg agtcgtatta g 331
<210> 64
   <211> 429
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (405)..(429)
   <223> Promoter
<400> 64
   ctaatacgac tcactatagg gcgatcagat gattggaacg gaggaaaatg tccaagtagc      60
   attcgtgggc tcgattgtcg agtgtcacaa gctcaaggtg tttactcagg aagaagcact     120
   gagattcctt gcggcaaaga tgaagcagcg gatgtttgga ccacagaaag cggaagaccc     180
   ccttgacaag gcatgggaag ccgtactttc atccgtagtc aaccatattc ccgttcaatc     240
   gcctgactac aatatgaccg tccgggcaca ctatcttgca ctaatggtgc gtcacatcat     300
   tcaggcgcgt tatgatcgcc gcttcattga cgatcgcgac tattacggca acaaacgaat     360
   tgagcttccg ggttcgatga tatcgctgct gtttgaagac ctgtttcgcc ctatagtgag     420
   tcgtattag                                                             429
<210> 65
   <211> 494
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (470)..(494)
   <223> Promoter
<400> 65
   ctaatacgac tcactatagg gcgaaatcaa ttcgtctgca gatctcaccg attttctgat      60
   atcgctggga gtccaggata ttcgactact atgcggagct gaattcagca aaacacacgt     120
   ctactatgta ttccacaacg gtgttattaa aggcgtcgtt gaggatcatc gcaggcttat     180
   caacgagatt cggcaatttc gtcggaaggg atacttgtcg ccttacttat cagtttatcc     240
   aaatcatcta catcgctgtg tgtatattgt aactgacggt ggtcgtttct gcaggccgtt     300
   tatcattgtt gaggatggtc agccaaaagt tacgcagaaa catttggacg acctcaaagc     360
   caatatatat aacttccaag acttcctgga catgggcttt gtagagtttc tcgatgtaaa     420
   tgaggaaaac gacgcgctta tcgccattta tgaaaaagat atcacaatca tcgccctata     480
   gtgagtcgta ttag                                                       494
<210> 66
   <211> 476
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (452)..(476)
   <223> Promoter
<400> 66
   ctaatacgac tcactatagg gcgattcata aatggcgata agcgcgtcgt tttcctcatt       60
   tacatcgaga aactctacaa agcccatgtc caggaagtct tggaagttat atatattggc      120
   tttgaggtcg tccaaatgtt tctgcgtaac ttttggctga ccatcctcaa caatgataaa      180
   cggcctgcag aaacgaccac cgtcagttac aatatacaca cagcgatgta gatgatttgg      240
   ataaactgat aagtaaggcg acaactatcc cttccgacga aattgccgaa tctcgttgat      300
   aagcctgcga tgatcctcaa cgacgccttt aataacaccg ttgtggaata catagtagac      360
   gtgtgttttg ctgaattcag ctccgcatag tagtcgaata tcctggactc ccagcgatat      420
   cagaaaatcg gtgagatctg cagacgaatt gatcgcccta tagtgagtcg tattag          476
<210> 67
   <211> 474
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (450)..(474)
   <223> Promoter
<400> 67
   ctaatacgac tcactatagg gcgatcaatt cgtctgcaga tctcaccgat tttctgatat 60
   cgctgggaat ccaggatatt cgactactat gcggagctga attcagcaaa acacacgtct 120
   actatgtatt ccacaacggt gttattaaag gcgtcgttga ggatcatcgc aggcttatca 180
   acgagattcg gcaatttcgt cggaagggat acttgtcgcc ttacttatca gtttatccaa 240
   atcatctaca tcgctgtgtg tatattgtaa ctgacggtgg tcgtttctgc aggccgttta 300
   tcattgttga ggatggtcag ccaaaagtta cgcagaaaca tttggacgac ctcaaagcca 360
   atatatataa cttccaagac ttcctggaca tgggctttgt agagtttctc gatgtaaatg 420
   aggaaaacga cgcgcttatc gccatttatg tcgccctata gtgagtcgta ttag 474
<210> 68
   <211> 426
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (402)..(426)
   <223> Promoter
<400> 68
   ctaatacgac tcactatagg gcgagttttg aacaaaatga gtgttgagcg cggatttaag 60
   gccggtgtag tatataaaac agaaacgatc aatttgcgta agttatctgg ggatgtggga 120
   gtccagacat cgtgcgtttt tggtcgaaag gcaggagatt ctgagttaca gaaatttgta 180
   gatgttgatg gcctgccata catcggcagc agggtagtac agggagatcc ggtatgtgca 240
   tatataaatt tgaccacggg acaactgaag actgtaaggt attactcgac cgagccagca 300
   atcgtgcatg aagtgaaaat tcttggtaat gattccggta cagacaccct ccaacaaatc 360
   cagttgacgt atcttattga tcgaacgcca aatgatcgga gatcgcccta tagtgagtcg 420
   tattag 426
<210> 69
   <211> 415
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (391)..(415)
   <223> Promoter
<400> 69
   ctaatacgac tcactatagg gcgaaatgag tgttgagcgc ggatttaagg ccggtgtagt      60
   atataaaaca gaaacgatca atttgcgtaa gttatctggg gatgtgggag tccagacatc     120
   gtgcgttttt ggtcgaaagg caggagattc tgagttacag aaatttgtag atgttgatgg     180
   cctgccatac atcggcagca gggtagtaca gggagatccg gtatgtgcat atataaattt     240
   gaccacggga caactgaaga ctgtaaggta ttactcgacc gagccagcaa tcgtgcatga     300
   agtgaaaatt cttggtaatg attccggtac agacaccctc caacaaatcc agttgacgta     360
   tcttgttgat cgaacgccaa atgatcggag atcgccctat agtgagtcgt attag          415
<210> 70
   <211> 414
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (390)..(414)
   <223> Promoter
<400> 70
   ctaatacgac tcactatagg gcgaaatgag tgttgagcgc ggatttaagg ccggtgtagt      60
   atataaaaca gaaacgatca atttgcgtaa gttatctggg gatgtgggag tccagacatc     120
   gtgcgttttt ggtcgaaagg caggagattc tgagttacag aaatttgtag atgttgatgg     180
   cctgccatac atcggcagca gggtagtaca gggagatccg gtatgtgcat atataaattt     240
   gaccacggga caactgaaga ctgtaaggta ttactcgacc gagccagcaa tcgtgcatga     300
   agtgaaaatt cttggtaatg attccggtac agacaccctc caacaaatcc agctgacgta     360
   tcttgttgat cgaacgccaa atgatcggag tcgccctata gtgagtcgta ttag           414
<210> 71
   <211> 316
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (292)..(316)
   <223> Promoter
<400> 71
   ctaatacgac tcactatagg gcgaaatggt ttctgctacc tgtgaggata gtatgcggga      60
   tgcttgtatt cgttttcttg cctcgaaagt caatctcaaa gcgcttgaca gtgagacaga     120
   gcttatgctc attgaagagg ccggcaaagt ggcagccctc gtcggtggag aggagtttgt     180
   gctgctggtt aagctcctca attcattaaa ggtagattgt acattttggc gtcttctcga     240
   acaagttaga atctatttag caaagtgcca atgtatcagc ttccaatacg catcgcccta     300
   tagtgagtcg tattag                                                     316
<210> 72
   <211> 311
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (287)..(311)
   <223> Promoter
<400> 72
   ctaatacgac tcactatagg gcgaattgga agctgataca ttggcacttt gctaaataga      60
   ttctaacttg ttcgagaaga cgccaaaatg tacaatctac ctttaatgaa ttgaggagct     120
   taaccagcag cacaaactcc tctccaccga cgagggctgc cactttgccg gcctcttcaa     180
   tgagcataag ctctgtctct ctgtcaagcg ctttgagatt gactttcgag gcaagaaaac     240
   gaatacaagc atcccgcata ctatcctcac aggtagcaga aaccatttcg ccctatagtg     300
   agtcgtatta g                                                          311
<210> 73
   <211> 311
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (287)..(311)
   <223> Promoter
<400> 73
   ctaatacgac tcactatagg gcgaattgga agctgataca ttggcacttt gctaaataga      60
   ttctaacttg ttcgagaaga cgccaaaatg tacaatctac ctttaatgaa ttgaggagct     120
   taaccagcag cacaaactcc tctccaccga cgagggctgc cactttgccg gcctcttcaa     180
   tgagcataag ctctgtctct ctgtcaagcg ctttgagatt gactttcgag gcaagaaaac     240
   gaatacaagc atcccgcata ctatcctcac aggtagcaga aaccatttcg ccctatagtg     300
   agtcgtatta g                                                          311
<210> 74
   <211> 327
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (303)..(327)
   <223> Promoter
<400> 74
   ctaatacgac tcactatagg gcgagatctt gttgaagccg gcttcttctt ccttggcatg      60
   cacgattaca cgaaatgctt ccattgcgac ggcggtctgt gtaattggga gacaggtgac     120
   gacccctggg tagagcatgc ccgctggttc cctgaatgtc aattcgttca gctaagcaag     180
   ggcggagcat tcatcgctga gtgccaacaa cgtcacgaaa aactagttaa tggcgcggta     240
   gcccaggcag aacttcaggc ttttagtgaa gtagaaccgg gaggaacagg cagtgactca     300
   aattcgccct atagtgagtc gtattag                                         327
<210> 75
   <211> 311
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (287)..(311)
   <223> Promoter
<400> 75
   ctaatacgac tcactatagg gcgaagtcac tgcctgttcc tcccggttct acttcactaa      60
   aagcctgaag ttctgcctgg gctaccgcgc cattaactag tttttcgtga cgttgttggc     120
   actcagcgat gaatgctccg cccttgctta gctgaacgaa ttgacattca gggaaccagc     180
   gggcatgctc tacccagggg tcgtcacctg tctcccaatt acacagaccg ccgtcgcaat     240
   ggaagcattt cgtgtaatcg tgcatgccaa ggaagaagaa gccggcttcg ccctatagtg     300
   agtcgtatta g                                                          311
<210> 76
   <211> 347
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (323)..(347)
   <223> Promoter
<400> 76
   ctaatacgac tcactatagg gcgattccgc ttcatttgag aactgagctt gaagaaataa      60
   tgcagtcgcc cgtcgtcaag ttctacctcg agaaaggtgt accgaaacaa gtgattcgaa     120
   tgaccgtaaa aaatatatgc ttgacaacga gcgcggtttc cgtgatcttg acgaaattac     180
   acacgtactc ggacaggtgc tcagcttcgg caacaagaag actgcgcctg ccaatgaaaa     240
   aggtaggtgg ataccggata tttgtcggga attcaatgca gctgaacccg atgaggttga     300
   ttcagaattg gcatacaata gaatcgccct atagtgagtc gtattag                   347
<210> 77
   <211> 332
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (308)..(332)
   <223> Promoter
<400> 77
   ctaatacgac tcactatagg gcgattccgc ttcatttgag aactgagctt gaagaaataa      60
   tgcagtcgcc cgtcgtcaag ttctacctcg agaaaggtgt accgaaacaa gtgattcgaa     120
   tgaccgtaaa aaaatatatg cttgacaacg agcgcggttt ccgtgatctt gacgaaatta     180
   cacacgtact cggacaggtg ctcagcttcg gcaacaagaa gactgcgcct gccaatgaaa     240
   aaggtaggtg gataccggat atttgtcggg aattcaatgc agctgaaccc gatgaggttg     300
   attcagaatc gccctatagt gagtcgtatt ag                                   332
<210> 78
   <211> 331
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (307)..(331)
   <223> Promoter
<400> 78
   ctaatacgac tcactatagg gcgattccgc ttcatttgag aactgagctt gaagaaataa      60
   tgcagtcgcc cgtcgtcaag ttctacctcg agaaaggtgt accgaaacaa gtgattcgaa     120
   tgaccgtgaa aaaatatatg cttgacaacg agcgcggttt ccgtgatctt gacgaaatta     180
   cacacgtact cggacaggtg ctcagcttcg gcaacaagaa gactgcgcct gccaatgaaa     240
   aaggtaggtg gataccggat atttgtcggg aattcaatgc agctgaaccc gatgaggttg     300
   attcagatcg ccctatagtg agtcgtatta g                                    331
<210> 79
   <211> 327
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (303)..(327)
   <223> Promoter
<400> 79
   ctaatacgac tcactatagg gcgatggcta attaatagta ggccgaagaa ctttttgagt      60
   ggcctcgata tgtccgacgt tgtggcttcg tgggaggttc ctttggttgg ccaagcttac     120
   cgagtcgaat tcgaacacgg aagtgcaacg ggtaaacgtg ttgtgtacgt taatggactc     180
   gaggtgttac gaaaacactg gctttttaag cttgttggcg aggaaagctt tgacatattg     240
   ggacataagt gcatcatttc tatcaaagcc gtaggaggct tcaggttggt agcaaactcc     300
   agttcgccct atagtgagtc gtattag                                          327
<210> 80
   <211> 325
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (301)..(325)
   <223> Promoter
<400> 80
   ctaatacgac tcactatagg gcgatggcta attaatagta ggccgaagaa ctttttgagt      60
   ggcctcgata tgtccgacgt tgtggcttcg tgggaggttc ctttggttgg ccaagcttac     120
   cgagtcgaat tcgaacacgg aagtgcaacg ggtaaacgtg ttgtgtacgt taatggactc     180
   gaggtgttac gaaaacactg gctttttaag cttgttggcg aggaaagctt tgacatattg     240
   ggacataagt gcatcatttc tatcaaagcc gtaggaggct tcaggttggt agcaaactcc     300
   atcgccctat agtgagtcgt attag                                           325
<210> 81
   <211> 325
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (301)..(325)
   <223> Promoter
<400> 81
   ctaatacgac tcactatagg gcgatggagt ttgctaccaa cctgaagcct cctacggctt      60
   tgatagaaat gatgcactta tgtcccaata tgtcaaagct ttcctcgcca acaagcttaa     120
   aaagccagtg ttttcgtaac acctcgagtc cattaacgta cacaacacgt ttacccgttg     180
   cacttccgtg ttcgaattcg actcggtaag cttggccaac caaaggaacc tcccacgaag     240
   ccacaacgtc ggacatatcg aggccactca aaaagttctt cggcctacta ttaattagcc     300
   atcgccctat agtgagtcgt attag                                           325
<210> 82
   <211> 331
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (307)..(331)
   <223> Promoter
<400> 82
   ctaatacgac tcactatagg gcgaggtctt gacaacacat gctaccctcg aacacgccga       60
   ctgcgtcttc atgatggaca atgaggccat ctatcagatc tgccgtcgga accttggagt      120
   cgagcgaccg gcgtaccaga atctcaaccg tctgatcagt caggccgttt cggcgattac      180
   cgcttctcta cgtttctccg gagcgctgaa tgttgatctt aacgagttcc aaactaattt      240
   agttccatac ccgcgaatcc attttcccct cgtcacttac gctccgatca tttctgctga      300
   gaaggcttcg ccctatagtg agtcgtatta g                                     331
<210> 83
   <211> 327
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (303)..(327)
   <223> Promoter
<400> 83
   ctaatacgac tcactatagg gcgaggtctt gacaacacat gctaccctcg aacacgccga       60
   ctgcgtcttc atgatggaca atgaggccat ctatcagatc tgccgtcgga accttggagt      120
   cgagcgaccg gcgtaccaga atctcaaccg tctgatcagt caggccgttt cggcgattac      180
   cgcttctcta cgtttctccg gagcgctgaa tgttgatctt aacgagttcc aaactaattt      240
   agttccatac ccgcgaatcc attttccccc cgtcacttac gctccgatca tttctgctga      300
   gaatcgccct atagtgagtc gtattag                                          327
<210> 84
   <211> 325
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (301)..(325)
   <223> Promoter
<400> 84
   ctaatacgac tcactatagg gcgactcagc agaaatgatc ggagcgtaag tgacgggggg      60
   aaaatggatt cgcgggtatg gaactaaatt agtttggaac tcgttaagat caacattcag     120
   cgctccggag aaacgtagag aagcggtaat cgccgaaacg gcctgactga tcagacggtt     180
   gagattctgg tacgccggtc gctcgactcc aaggttccga cggcagatct gatagatggc     240
   ctcattgtcc atcatgaaga cgcagtcggc gtgttcgagg gtagcatgtg ttgtcaagac     300
   ctcgccctat agtgagtcgt attag                                           325
<210> 85
   <211> 380
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (356)..(380)
   <223> Promoter
<400> 85
   ctaatacgac tcactatagg gcgacaacgc tgtgcttcac gtagactcca cgttcgaaaa      60
   tgtcgactgc acgtttatgg ttgataatca aacactcttc aagctttgtc gagaccggct     120
   aaagattagg agtccatctt atgacaacgc aaatgctgtc atttcccagg gtttttcgtc     180
   aatcatgaat tcggtggggc tggatggatc cttgaatgtg gacctcagcg agttccaaac     240
   aaatctcgtc ccttttggaa gattacattt tacgatgatg agctacagtc cattcgttac     300
   atccggacac cgcgatctaa gccgtgagac gtccgtcgtg gagattactc gtgacctcgc     360
   cctatagtga gtcgtattag                                                 380
<210> 86
   <211> 378
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (354)..(378)
   <223> Promoter
<400> 86
   ctaatacgac tcactatagg gcgacaacgc tgtgcttcac gtagactcca cgttcgaaaa      60
   tgtcgactgc acgtttatgg ttgataatca aacactcctc aagctttgtc gagaccggct     120
   aaaggttagg agtccatctt atgacaacgc aaatgctgtc atttcccagg gtttttcgtc     180
   aatcatgaat tcggtggggc tggatggatc cttgaatgtg gacctcagcg agttccaaac     240
   aagtctcgtc ccttttggaa gattacattt tacgatgatg agctacagtc cattcgttac     300
   atccggacac cgcgatctaa gccgtgagac gtccgtcgtg gagattactc gtgatcgccc     360
   tatagtgagt cgtattag                                                   378
<210> 87
   <211> 377
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (353)..(377)
   <223> Promoter
<400> 87
   ctaatacgac tcactatagg gcgaaacgct gtgcttcacg tagactccac gttcgaaaat      60
   gtcgactgca cgtttatggt tgataatcaa acactcctca agctttgtcg agaccggcta     120
   aaggttagga gtccatctta tgacaacgca aatgctgtca tttcccaggg tttttcgtga     180
   atcatgaatt cggtggggct ggatggatcc ttgaatgtgg acctcagcga gttccaaaca     240
   agtctcgtcc cttttggaag attacatttt acgatgatga gctacagtcc attcgttaca     300
   tccggacacc gcgatctaag ccgtgagacg tccgtcgtgg agattactcg tgatcgccct     360
   atagtgagtc gtattag                                                    377
<210> 88
   <211> 463
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (439)..(463)
   <223> Promoter
<400> 88
   ctaatacgac tcactatagg gcgatatgga gaacatcgca caggacttcg gtaaaaagtg 60
   ccgattgggc ttcgccatct acccggctcc gcaggtttcc actgccgttg tcgaaccata 120
   caactcggtt ttgacgacac atgccaccct cgaacacgct gactgcgtat tcatgatgga 180
   taatgaggcg atctatcaga tctgtcgtcg gaatcttgga gttgaacgac cggcgtatca 240
   aaatctcaat cgactgatta gccaggccgt ttcggcgata accgcttctc tacgtttttc 300
   cggagcgttg aatgttgacc tcaacgaatt tcagacgaat ctcgtcccct acccgcgaat 360
   ccatttcccg ctcgtcactt atgctccgat tatttcggct gagaaggctc atcacgagca 420
   acataacgta ctggaaatct cgccctatag tgagtcgtat tag 463
<210> 89
   <211> 460
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (436)..(460)
   <223> Promoter
<400> 89
   ctaatacgac tcactatagg gcgaatggag aacatcgcac aggacttcgg taaaaagtgc      60
   cgattgggct tcgccatcta cccggctccg caggtttcca ctgccgttgt cgaaccatac     120
   aactcggttt tgacgacaca tgccaccctc gaacacgctg actgcgtatt catgatggat     180
   aatgaggcga tctatcagat ctgtcgtcgg aatcttggag ttgaacgacc ggcgtatcaa     240
   aatctcaatc gactgattag ccaggccgtt tcggcgataa ccgcttctct acgtttttcc     300
   ggagcgttga atgttgacct caacgaattt cagacgaatc tcgtccccta cccgcgaatc     360
   catttcccgc tcgtcactta tgctccgatt atttcggctg agaaggctca tcacgagcaa     420
   cataacgtac tggaaatcgc cctatagtga gtcgtattag                           460
<210> 90
   <211> 459
   <212> DNA
   <213> Artificial sequence
<220>
   <223> A double stranded RNA expressing construct targeting a Varroa gene
<220>
   <221> misc_feature
   <222> (1)..(24)
   <223> Promoter
<220>
   <221> misc_feature
   <222> (435)..(459)
   <223> Promoter
<400> 90
   ctaatacgac tcactatagg gcgaatggag aacatcgcac aggacttcgg taaaaagtgc      60
   cgattgggct tcgccatcta cccggctccg caggtttcca ctgccgttgt cgaaccatac     120
   aactcggttt tgacgacaca tgccaccctc gaacacgctg actgcgtatt catgatggat     180
   aatgaggcga tctatcagat ctgtcgtcgg aatcttggag ttgaacgacc ggcgtatcaa     240
   aatctcaatc gactgattag ccaggccgtt tcggcgataa ccgcttctct acgtttttcc     300
   ggagcgttga atgttgacct caacgaattt cagacgaatc tcgtccccta cccgcgaatc     360
   catttcccgc tcgtcactta tgctccgatt atttcggctg agaaggctca tcacgagcaa     420
   cataacgtac tggaatcgcc ctatagtgag tcgtattag                            459
<210> 91
   <211> 480
   <212> DNA
   <213> Artificial sequence
<220>
   <223> A double stranded RNA expressing construct targeting GFP
<220>
   <221> misc_feature
   <222> (1)..(24)
   <223> Promoter
<220>
   <221> misc_feature
   <222> (456)..(480)
   <223> Promoter
<400> 91
   ctaatacgac tcactatagg gcgagccaac acttgtcact actttcggtt atggtgttca      60
   atgctttgcg agatacccag atcatatgaa acagcatgac tttttcaaga gtgccatgcc     120
   tgaaggttat gtacaggaaa gaactatatt tttcaaagat gacgggaact acaagacacg     180
   tgctgaagtc aagtttgaag gtgataccct tgttaataga atcgagttaa aaggtattga     240
   ttttaaagaa gatggaaaca ttcttggaca caaattggaa tacaactata actcacacaa     300
   tgtatacatc atggcagaca aacaaaagaa tggaatcaaa gttaacttca aaattagaca     360
   caacattgaa gatggaagcg ttcaactagc agaccattat caacaaaata ctccaattgg     420
   cgatggccct gtccttttac cagacaacca ttaccttcgc cctatagtga gtcgtattag     480

## Description

### FIELD AND BACKGROUND OF THE INVENTION

The invention relates to compositions for controlling *Varroa* mite infestation in bees.

Honey bees, Apis mellifera, are required for the effective pollination of crops and are therefore critical to world agriculture. Honey bees also produce economically important products, including honey and bees wax. Honey bees are susceptible to a number of parasites and pathogens, including the ectoparasitic mite, Varroa destructor.

Colony Collapse Disorder (CCD) of honeybees is threatening to annihilate U.S. and world agriculture. Indeed, in the recent outbreak of CCD in the U.S in the winter of 2006-2007, an estimated 25 % or more of the 2.4 million honeybee hives were lost because of CCD. An estimated 23 % of beekeeping operations in the United States suffered from CCD over the winter of 2006-2007, affecting an average of 45 % of the beekeepers operations. In the winter of 2007-2008, the CCD action group of the USDA-ARS estimated that a total of 36 % of all hives from commercial operations were destroyed by CCD.

CCD is characterized by the rapid loss from a colony of its adult bee population, with dead adult bees usually found at a distance from the colony. At the final stages of collapse, a queen is attended only by a few newly emerged adult bees. Collapsed colonies often have considerable capped brood and food reserves. The phenomenon of CCD was first reported in 2006; however, beekeepers noted unique colony declines consistent with CCD as early as 2004. Various factors such as mites and infectious agents, weather patterns, electromagnetic (cellular antennas) radiation, pesticides, poor nutrition and stress have been postulated as causes. To date, control of CCD has focused on Varroa mite control, sanitation and removal of affected hives, treating for opportunistic infections (such as *Nosema*) and improved nutrition. No effective preventative measures have been developed to date.

Varroa mites parasitize pupae and adult bees and reproduce in the pupal brood cells. The mites use their mouths to puncture the exoskeleton and feed on the bee's hemolymph. These wound sites in the exoskeleton harbor bacterial infections, such as Melissococcus pluton, which causes European foulbrood. In addition, to their parasitic effects, Varroa mites are suspected of acting as vectors for a number of honey bee pathogens, including deformed wing virus (DWV), Kashmir bee virus (KBV), acute bee paralysis virus (ABPV) and black queen cell virus (BQCV), and may weaken the immune systems of their hosts, leaving them vulnerable to infections. If left untreated Varroa infestations typically result in colony-level mortality.

Current methods of treating Varroa infestations are proving to be ineffective as the mites develop resistance to existing miticides. In addition, the use of such miticides may introduce injurious chemicals into honey that is intended for human consumption.

U.S. Patent Application 20090118214 teaches the use of dsRNA for prevention and treatment of viral infections in honeybees.

### SUMMARY OF THE INVENTION

According to the present invention there is provided a bee-ingestible composition comprising at least one nucleic acid agent comprising a nucleic acid sequence which downregulates expression of a Varroa destructor mite-specific mRNA for use in preventing or treating a Varroa destructor mite infestation of a bee hive, wherein said mRNA encodes a polypeptide selected from the group consisting of ATPase subunit A, RNA polymerase I, RNA polymerase III, Inhibitor of apoptosis (IAP), FAS apoptotic inhibitor and α-Tubulin, wherein said agent is a double stranded RNA and wherein the double stranded RNA is at least 100 bases long and does not comprise any sequence longer than 19 bases entirely homologous to any bee-genome sequence or human-genome RNA sequence.

According to a preferred embodiment of the invention, the nucleic acid agents are as set forth in SEQ ID Nos: 1, 13, 27, 30 and 39.

According to a preferred embodiment of the invention, the nucleic acid agents are as set forth in SEQ ID Nos: 1, 4, 7, 10, 13, 16, 19, 22, 25, 27, 30, 33, 36 and 39.

According to a preferred embodiment of the invention, the composition is in solid form.

According to a preferred embodiment of the invention, the composition is in liquid form.

According to a preferred embodiment of the invention, the composition comprises protein.

According to a preferred embodiment of the invention, the protein is in the form of pollen and/or soy patties.

According to a preferred embodiment of the invention, the liquid is a sucrose solution.

According to a preferred embodiment of the invention, the liquid is a corn syrup solution.

According to a preferred embodiment of the invention, the liquid further comprises a carbohydrate or sugar supplement.

According to a preferred embodiment of the invention, the bee is a honeybee.

According to a preferred embodiment of the invention, the honeybee is a forager.

According to a preferred embodiment of the invention, the honeybee is a hive bee.

According to a preferred embodiment of the invention, the honeybee is a bee of a colony.

Unless otherwise defined, all technical and/or scientific terms used herein have the same meaning as commonly understood by one of ordinary skill in the art to which the invention pertains.

### BRIEF DESCRIPTION OF THE DRAWINGS

Some embodiments of the invention are herein described, by way of example only, with reference to the accompanying drawings. In this regard, the description taken with the drawings makes apparent to those skilled in the art how embodiments of the invention may be practiced.

In the drawings:

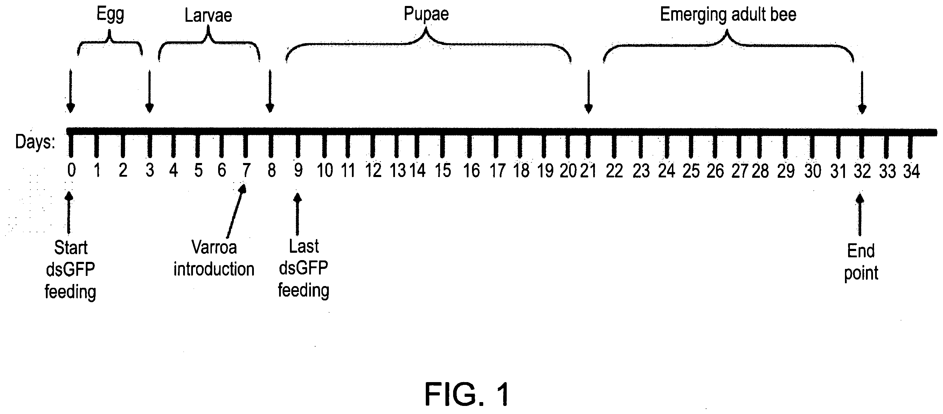

FIG.1 is a schematic representation of the time-course of various experiment for dsRNA transfer to Varroa mites.

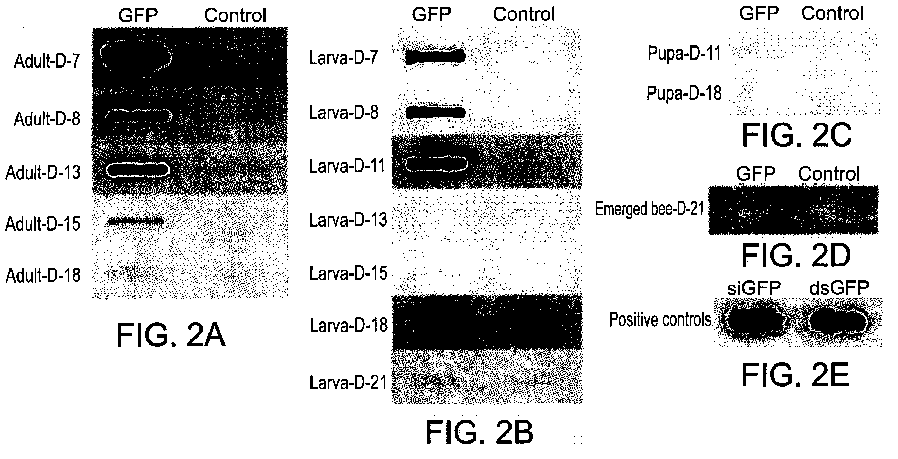

FIGs. 2A-E are photographs of the results of Slot blot analysis of the presence of dsRNA-GFP in ingested bees (Figure 2A), in larvae fed by adult bees (Figure 2B), in pupae (Figure 2C), and in the newly-emerge bees (Figure 2D). The presence of dsRNA-GFP and of siRNA derived from it was analyzed by Northern blots (Figure 2E). D=days after administration of dsRNA-GFP to the hive.

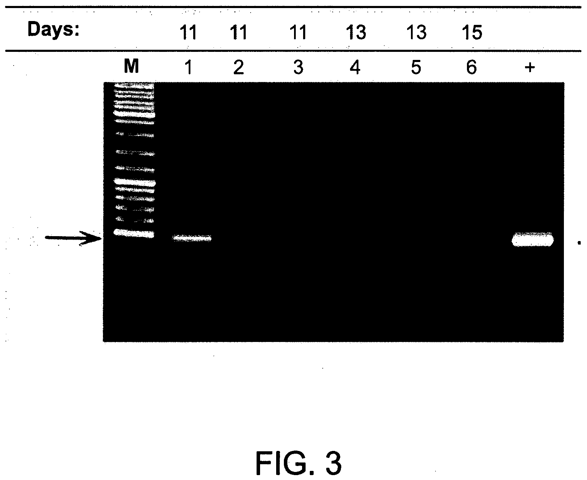

FIG. 3 is a photograph illustrating the results of RT-PCR analysis of Varroa-extracted RNA at the days indicated in the top row (time as indicated in Figure 1). Green numbers (top row) indicate Varroa individuals which had been placed on dsRNA-GFP-ingested bees and black numbers indicate RNA from Varroa placed on control bees. + = positive control (a GFP-carrying plasmid).

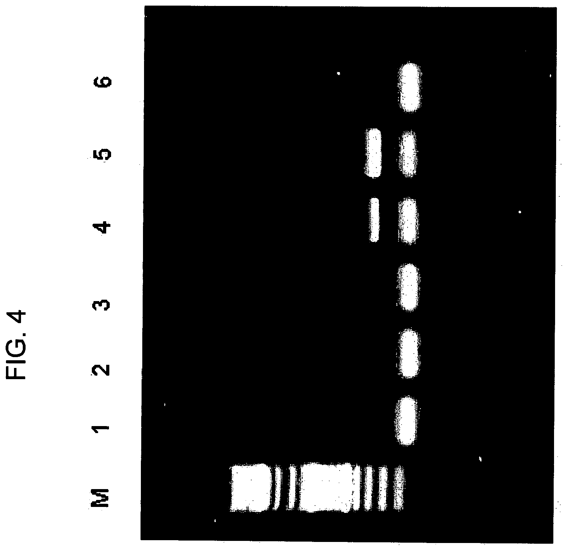

FIG. 4 is a photograph illustrating RT-PCR of Varroa RNA with primers to apoptosis inhibitor protein (IAP; sequence 27). M: size markers. Lanes 1-3: Template RNA of Varroa from hives treated with dsRNA of sequences 27. Lane 4: Template RNA of Varroa from control hives. Lane 5: Positive control (a IAP-carrying plasmid). Lane 6: Negative control (no template).

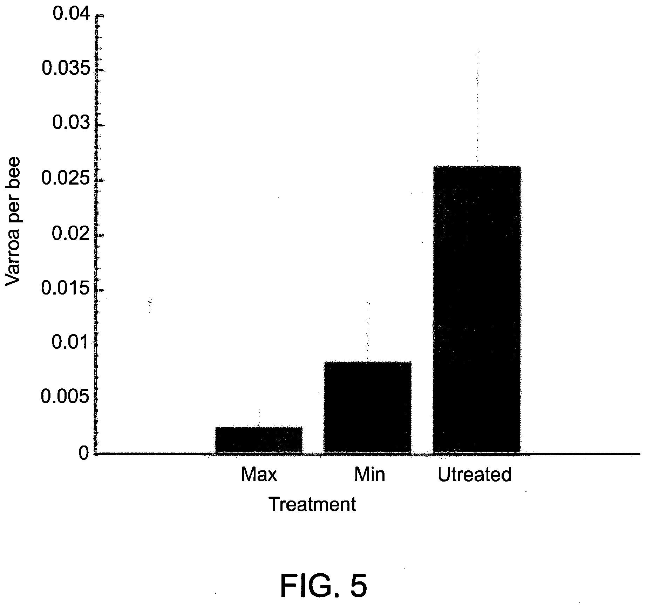

FIG. 5 is a bar graph illustrating the Varroa count per bee (adult bees plus larvae inside sealed cells) in control hives and in hives treated with dsRNA mixture I (Min) and with dsRNA mixture II (Max).

### DESCRIPTION OF EMBODIMENTS OF THE INVENTION

The present invention relates to compositions for reducing the susceptibility of bees to Varroa mite infestation.

Bees are susceptible to a myriad of viral infections. Treatment of such infections by down-regulation of a particular viral gene product has shown to be successful in eliminating virally induced infections in the bee (see U.S. Patent Application 20090118214).

The present inventors now propose treatment of Varroa mite infestations in bees by down-regulating particular Varroa mite gene products.

Varroa mites parasitize pupae and adult bees and reproduce in the pupal brood cells. The mites use their mouths to puncture the exoskeleton and feed on the bee's hemolymph. The present inventors unexpectedly found that polynucleotide agents administered to the bees to treat Varroa mite infestations presented in the bee's hemolymph thereby becoming available to the mite.

The present inventors have shown that dsRNA can successfully be transferred to Vorroa mites (Figures 2A-E), that the dsRNA can serve to down-regulate expression of a particular gene in the Varroa mite (Figure 4) and further that targeting of particular genes for down-regulation can result in a reduction in the number of Varroa mites (Figure 5)

Thus, disclosed is provided a method of preventing or treating a Varroa destructor mite infestation of a bee, the method comprising administering to the bee an effective amount of a nucleic acid agent comprising a nucleic acid sequence which downregulates expression of a gene product of a Varroa destructor mite, thereby preventing or treating a Varroa destructor mite infestation of a bee.

As used herein, the term "bee" refers to both an adult bee and pupal cells thereof. According to one embodiment, the bee is in a hive.

An adult bee is defined as any of several winged, hairy-bodied, usually stinging insects of the superfamily Apoidea in the order Hymenoptera, including both solitary and social species and characterized by sucking and chewing mouthparts for gathering nectar and pollen. Exemplary bee species include Apis, Bombus, Trigona and Osmia. In one embodiment, bees include bumblebees (Bombus terrestris), honeybees (Apis mellifera) (including foragers and hive bees) and Apis cerana.

The bee is part of a colony.

The term "colony" refers to a population of bees comprising dozens to typically several tens of thousand bees that cooperate in nest building, food collection, and brood rearing. A colony normally has a single queen, the remainder of the bees being either "workers" (females) or "drones" (males). The social structure of the colony is maintained by the queen and workers and depends on an effective system of communication. Division of labor within the worker caste primarily depends on the age of the bee but varies with the needs of the colony. Reproduction and colony strength depend on the queen, the quantity of food stores, and the size of the worker force. Honeybees can also be subdivided into the categories of "hive bees", usually for the first part of a workers lifetime, during which the "hive bee" performs tasks within the hive, and "forager bee", during the latter part of the bee's lifetime, during which the "forager" locates and collects pollen and nectar from outside the hive, and brings the nectar or pollen into the hive for consumption and storage.

According to the present invention the agents comprised in the composition of the present invention are used to prevent the Varroa destructor mite from living as a parasite on the bee, or larvae thereof.

The phrase "Varroa destructor mite" refers to the external parasitic mite that attacks honey bees Apis cerana and Apis mellifera. The mite may be at an adult stage, feeding off the bee, or at a larval stage, inside the honey bee brood cell.

As mentioned, the agents are capable of downregulating expression of a gene product of a Varroa destructor mite.

As used herein, the phrase "gene product" refers to an RNA molecule or a protein.

The Varroa destructor mite gene product is one which is essential for mite viability. Down-regulation of such a gene product would typically result in killing of the Varroa mite. According to another embodiment, the Varroa destructor mite gene product is one which is essential for mite reproduction. Down-regulation of such a gene product would typically result in the prevention of reproduction of the Varroa mite and the eventual extermination of the mite population. According to yet another embodiment, the Varroa destructor mite gene product is one which is required to generate pathogenic symptoms in the bee.

Exemplary gene products that may be down-regulated according to this aspect of the present invention include NADH dehydrogenase; subunit 2- Genbank accession NC_004454; ATP synthetase; subunit 8 - NC_004454; ATP synthetase; subunit 6 - NC_004454; sodium channel gene - Genbank accession No. FJ216963; Cytochrome oxydase subunit I - Genbank accession No. EF025469.

It will be appreciated that whilst the agents of the present invention are capable of downregulating expression of a gene product of a Varroa destructor mite, it is preferable that they downregulate to a lesser extent expression of the gene product in other animals, such as the bee. Accordingly, the agents comprised in the composition of the present invention must be able to distinguish between the mite gene and the bee gene, down-regulating the former to a greater extent than the latter. According to another embodiment the agents do not down-regulate the bee gene whatsoever. This may be effected by targeting a gene that is expressed differentially in the mite and not in the bee e.g. the mite sodium channel gene - FJ216963. Alternatively, the agents may be targeted to mite-specific sequences of a gene that is expressed both in the mite and in the bee.

The agents target segments of Varroa genes that are at least 100 bases long and do not carry any sequence longer than 19 bases that is entirely homologous to any bee-genome sequence or human-genome sequence.

Examples of such gene segments are provided herein below:
SEQ ID NO: 1. Varroa gene homologous to ATPase subunit A (segment 1); SEQ ID NO: 2. Varroa gene homologous to ATPase subunit A (segment 2); SEQ ID NO: 3. Varroa gene homologous to ATPase subunit A (segment 3); SEQ ID NO: 4. Varroa gene homologous to ATPase subunit A (segment 4); SEQ ID NO: 5. Varroa gene homologous to ATPase subunit A (segment 5); SEQ ID NO: 6. Varroa gene homologous to ATPase subunit A (segment 6); SEQ ID NO: 7. Varroa gene homologous to ATPase subunit A (segment 7); SEQ ID NO: 8. Varroa gene homologous to ATPase subunit A (segment 8); SEQ ID NO: 9. Varroa gene homologous to ATPase subunit A (segment 9); SEQ ID NO: 10. Varroa gene homologous to RNA polymerase I (segment 1); SEQ ID NO: 11. Varroa gene homologous to RNA polymerase I (segment 2); SEQ ID NO: 12. Varroa gene homologous to RNA polymerase I (segment 3); SEQ ID NO: 13. Varroa gene homologous to RNA polymerase III (segment 1); SEQ ID NO: 14. Varroa gene homologous to RNA polymerase III (segment 2); SEQ ID NO: 15. Varroa gene homologous to RNA polymerase III (segment 3); SEQ ID NO: 16. Varroa gene homologous to RNA polymerase III (segment 4); SEQ ID NO: 17. Varroa gene homologous to RNA polymerase III (segment 5); SEQ ID NO: 18. Varroa gene homologous to RNA polymerase III (segment 6); SEQ ID NO: 19. Varroa gene homologous to RNA polymerase III (segment 7) SEQ ID NO: 20. Varroa gene homologous to RNA polymerase III (segment 8); SEQ ID NO: 21. Varroa gene homologous to RNA polymerase III (segment 9); SEQ ID NO: 22. Varroa gene homologous to toInhibitor of apoptosis (IAP; segment 1); SEQ ID NO: 23. Varroa gene homologous to to Inhibitor of apoptosis (IAP; segment 2); SEQ ID NO: 24. Varroa gene homologous to to Inhibitor of apoptosis (IAP; segment 3); SEQ ID NO: 25. Varroa gene homologous to to Inhibitor of apoptosis (IAP; segment 4); SEQ ID NO: 26. Varroa gene homologous to to Inhibitor of apoptosis (IAP; segment 5); SEQ ID NO: 27. Varroa gene homologous to Inhibitor of apoptosis (IAP; segment 6); SEQ ID NO: 28. Varroa gene homologous to Inhibitor of apoptosis (IAP; segment 7); SEQ ID NO: 29. Varroa gene homologous to Inhibitor of apoptosis (IAP; segment 8); SEQ ID NO: 30. Varroa gene homologous to FAS apoptotic inhibitor (segment 1); SEQ ID NO: 31. Varroa gene homologous to FAS apoptotic inhibitor (segment 2); SEQ ID NO: 32. Varroa gene homologous to FAS apoptotic inhibitor (segment 3); SEQ ID NO: 33. Varoa gene homologous to α-Tubulin (segment 1); SEQ ID NO: 34. Varoa gene homologous to α-Tubulin (segment 2); SEQ ID NO: 35. Varoa gene homologous to α-Tubulin (segment 3); SEQ ID NO: 36. Varoa gene homologous to α-Tubulin (segment 4); SEQ ID NO: 37. Varoa gene homologous to α-Tubulin (segment 5); SEQ ID NO: 38. Varoa gene homologous to α-Tubulin (segment 6); SEQ ID NO: 39. Varoa gene homologous to α-Tubulin (segment 7); SEQ ID NO: 40. Varoa gene homologous to α-Tubulin (segment 8); SEQ ID NO: 41. Varoa gene homologous to α-Tubulin (segment 9); SEQ ID NO: 42.NADH dehydrogenase; subunit 2 (NC_004454): bases 709 to 974; SEQ ID NO: 43. ATP synthetase; subunit 8 (NC_004454): bases 3545 to 3643; SEQ ID NO: 44. Sodium channel protein (AY259834): bases 3336-3836.

It will be appreciated that more than one gene may be targeted in order to maximize the cytotoxic effect on the Varroa mites.

Thus, according to one embodiment, the following group of genes are targeted - ATPase subunit A, RNA polymerase III, Inhibitor of apoptosis (IAP), FAS apoptotic inhibitor and α-Tubulin (e.g. using nucleic acid agents having the sequence as set forth in 1, 13, 27, 30 and 39).

According to another embodiment, the following group of genes are targeted - ATPase subunit A, RNA polymerase I, RNA polymerase III, Inhibitor of apoptosis (IAP), FAS apoptotic inhibitor and α-Tubulin.

It will be appreciated that as well as down-regulating a number of genes, the present invention further contemplates using a number of agents to down-regulate the same gene (e.g. a number of dsRNAs each hybridizing to a different segment of the same gene). Thus, for example, the present inventors showed maximal cytotoxic activity when the following mixture of dsRNAs was used: SEQ ID Nos:1, 4, 7, 10, 13, 16, 19, 22, 25, 27, 30, 33, 36 and 39 and less of a cytotoxic activity when the following mixture of dsRNAs was used: SEQ ID Nos: 1, 13, 27, 30 and 39.

Tools which are capable of identifying species-specific sequences may be used for this purpose - e.g. BLASTN and other such computer programs

As used herein, the term "downregulating expression" refers to causing, directly or indirectly, reduction in the transcription of a desired gene, reduction in the amount, stability or translatability of transcription products (e.g. RNA) of the gene, and/or reduction in translation of the polypeptide(s) encoded by the desired gene. Downregulating expression of a gene product of a Varroa destructor mite can be monitored, for example, by direct detection of gene transcripts (for example, by PCR), by detection of polypeptide(s) encoded by the gene or bee pathogen RNA (for example, by Western blot or immunoprecipitation), by detection of biological activity of polypeptides encode by the gene (for example, catalytic activity and ligand binding), or by monitoring changes in the Vaarroa destructor mite (for example, reduced proliferation of the mite, reduced virulence of the mite and reduced motility of the mite) and by testing bee infectivity/pathogenicity.

Downregulation of a Varroa destructor mite gene product can in principle be effected on the genomic and/or the transcript level using a variety of agents which interfere with transcription and/or translation (e.g., RNA silencing agents, Ribozyme, DNAzyme and antisense).

According to the present invention, the agent which down-regulates expression of a Varroa destructor mite gene product is a polynucleotide agent, namely an RNA silencing agent that is a double stranded RNA (dsRNA) which is at least 100 bases long and does not comprise any sequence longer than 19 bases entirely homologous to any bee-genome sequence or human-genome RNA sequence.

The presence of long dsRNAs in cells stimulates the activity of a ribonuclease III enzyme referred to as dicer. Dicer is involved in the processing of the dsRNA into short pieces of dsRNA known as short interfering RNAs (siRNAs). Short interfering RNAs derived from dicer activity are typically about 21 to about 23 nucleotides in length and comprise about 19 base pair duplexes. The RNAi response also features an endonuclease complex, commonly referred to as an RNA-induced silencing complex (RISC), which mediates cleavage of single-stranded RNA having sequence complementary to the antisense strand of the siRNA duplex. Cleavage of the target RNA takes place in the middle of the region complementary to the antisense strand of the siRNA duplex.

The dsRNA is at least 100 bp. The use of long dsRNAs can provide numerous advantages in that the cell can select the optimal silencing sequence alleviating the need to test numerous siRNAs; long dsRNAs will allow for silencing libraries to have less complexity than would be necessary for siRNAs; and, perhaps most importantly, long dsRNA could prevent viral escape mutations when used as therapeutics.

Various studies demonstrate that long dsRNAs can be used to silence gene expression without inducing the stress response or causing significant off-target effects - see for example [Strat et al., Nucleic Acids Research, 2006, Vol. 34, No. 13 3803-3810; Bhargava A et al. Brain Res. Protoc. 2004;13:115-125; Diallo M., et al., Oligonucleotides. 2003;13:381-392; Paddison P.J., et al., Proc. Natl Acad. Sci. USA. 2002;99:1443-1448; Tran N., et al., FEBS Lett. 2004;573:127-134].

The synthesis of RNA silencing agents suitable for use with the present invention can be effected as follows. First, the Varroa mite target mRNA is scanned downstream of the AUG start codon for AA dinucleotide sequences. Occurrence of each AA and the 3' adjacent 19 nucleotides is recorded as potential siRNA target sites. Preferably, siRNA target sites are selected from the open reading frame, as untranslated regions (UTRs) are richer in regulatory protein binding sites. UTR-binding proteins and/or translation initiation complexes may interfere with binding of the siRNA endonuclease complex [Tuschl ChemBiochem. 2:239-245]. It will be appreciated though, that siRNAs directed at untranslated regions may also be effective, as demonstrated for GAPDH wherein siRNA directed at the 5' UTR mediated about 90 % decrease in cellular GAPDH mRNA and completely abolished protein level (wwwdotambiondotcom/techlib/tn/91/912dothtml).

Second, potential target sites are compared to an appropriate genomic database (e.g., human, bee, mouse, rat etc.) using any sequence alignment software, such as the BLAST software available from the NCBI server (wwwdotncbidotnlmdotnihdotgov/BLAST/). Putative target sites which exhibit significant homology to other coding sequences are filtered out. Qualifying target sequences are selected as template for siRNA synthesis. Preferred sequences are those including low G/C content as these have proven to be more effective in mediating gene silencing as compared to those with G/C content higher than 55 %.

Several target sites are preferably selected along the length of the target gene or sequence for evaluation. For better evaluation of the selected siRNAs, a negative control is preferably used in conjunction. Negative control siRNA preferably include the same nucleotide composition as the siRNAs but lack significant homology to the genome. Thus, a scrambled nucleotide sequence of the siRNA is preferably used, provided it does not display any significant homology to any other gene or bee pathogen target sequence.

A siRNA that may be used in this aspect of the present invention is one which targets a mite-specific gene. Exemplary siRNAs are provided in SEQ ID NOs: 45-47.
SEQ ID NO: 45: attttattcaattaaagtatt
SEQ ID NO: 46: atacctcaaatgtatccttca
SEQ ID NO: 47: ggccaatcccgattccggcga

It will be appreciated that the RNA silencing agent of the present invention need not be limited to those molecules containing only RNA, but further encompasses chemically-modified nucleotides and non-nucleotides.

The RNA silencing agent provided herein can be functionally associated with a cell-penetrating peptide. As used herein, a "cell-penetrating peptide" is a peptide that comprises a short (about 12-30 residues) amino acid sequence or functional motif that confers the energy-independent (i.e., non-endocytotic) translocation properties associated with transport of the membrane-permeable complex across the plasma and/or nuclear membranes of a cell. The cell-penetrating peptide used in the membrane-permeable complex of the present invention preferably comprises at least one non-functional cystein residue, which is either free or derivatized to form a disulfide link with a double-stranded ribonucleic acid that has been modified for such linkage. Representative amino acid motifs conferring such properties are listed in U.S. Pat. No. 6,348,185. The cell-penetrating peptides of the present invention preferably include, but are not limited to, penetratin, transportan, pIsl, TAT(48-60), pVEC, MTS, and MAP.

The polynucleotide down-regulating agents of the present invention may be generated according to any polynucleotide synthesis method known in the art such as enzymatic synthesis or solid phase synthesis. Equipment and reagents for executing solid-phase synthesis are commercially available from, for example, Applied Biosystems. Any other means for such synthesis may also be employed; the actual synthesis of the polynucleotides is well within the capabilities of one skilled in the art and can be accomplished via established methodologies as detailed in, for example, "Molecular Cloning: A laboratory Manual" Sambrook et al., (1989); "Current Protocols in Molecular Biology" Volumes I-III Ausubel, R. M., ed. (1994);.Ausubel et al., "Current Protocols in Molecular Biology", John Wiley and Sons, Baltimore, Maryland (1989); Perbal, "A Practical Guide to Molecular Cloning", John Wiley & Sons, New York (1988) and "Oligonucleotide Synthesis" Gait, M. J., ed. (1984) utilizing solid phase chemistry, e.g. cyanoethyl phosphoramidite followed by deprotection, desalting and purification by for example, an automated trityl-on method or HPLC.

The polynucleotide agents of the present invention may comprise heterocylic nucleosides consisting of purines and the pyrimidines bases, bonded in a 3' to 5' phosphodiester linkage. Preferably used polynucleotide agents are those modified in either backbone, internucleoside linkages or bases, as is broadly described hereinunder.

Specific examples of preferred polynucleotide agents useful according to this aspect of the present invention include polynucleotide agents containing modified backbones or non-natural internucleoside linkages. Polynucleotide agents having modified backbones include those that retain a phosphorus atom in the backbone, as disclosed in U.S. Pat. NOs: 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466, 677; 5,476,925; 5,519,126; 5,536,821; 5,541,306; 5,550,111; 5,563,253; 5,571,799; 5,587,361; and 5,625,050.

Preferred modified polynucleotide backbones include, for example, phosphorothioates, chiral phosphorothioates, phosphorodithioates, phosphotriesters, aminoalkyl phosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms can also be used.

Alternatively, modified polynucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts, as disclosed in U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623, 070; 5,663,312; 5,633,360; 5,677,437; and 5,677,439.

Other polynucleotide agents which can be used according to the present invention, are those modified in both sugar and the internucleoside linkage, i.e., the backbone, of the nucleotide units are replaced with novel groups. The base units are maintained for complementation with the appropriate polynucleotide target. An example for such an polynucleotide mimetic, includes peptide nucleic acid (PNA). A PNA polynucleotide refers to a polynucleotide where the sugar-backbone is replaced with an amide containing backbone, in particular an aminoethylglycine backbone. The bases are retained and are bound directly or indirectly to aza nitrogen atoms of the amide portion of the backbone. United States patents that teach the preparation of PNA compounds include U.S. Pat. Nos. 5,539,082; 5,714,331; and 5,719,262. Other backbone modifications, which can be used in the present invention are disclosed in U.S. Pat. No: 6,303,374.

Polynucleotide agents of the present invention may also include base modifications or substitutions. As used herein, "unmodified" or "natural" bases include the purine bases adenine (A) and guanine (G), and the pyrimidine bases thymine (T), cytosine (C) and uracil (U). Modified bases include but are not limited to other synthetic and natural bases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Further bases include those disclosed in U.S. Pat. No: 3,687,808, those disclosed in The Concise Encyclopedia Of Polymer Science And Engineering, pages 858-859, Kroschwitz, J. I., ed. John Wiley & Sons, 1990, those disclosed by Englisch et al., Angewandte Chemie, International Edition, 1991, 30, 613, and those disclosed by Sanghvi, Y. S., Chapter 15, Antisense Research and Applications, pages 289-302, Crooke, S. T. and Lebleu, B. , ed., CRC Press, 1993. Such bases are particularly useful for increasing the binding affinity of the oligomeric compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2°C. [Sanghvi YS et al. (1993) Antisense Research and Applications, CRC Press, Boca Raton 276-278] and are presently preferred base substitutions, even more particularly when combined with 2'-O-methoxyethyl sugar modifications.

Following synthesis, the polynucleotide agents of the present invention may optionally be purified. For example, polynucleotides can be purified from a mixture by extraction with a solvent or resin, precipitation, electrophoresis, chromatography, or a combination thereof. Alternatively, polynucleotides may be used with no, or a minimum of, purification to avoid losses due to sample processing. The polynucleotides may be dried for storage or dissolved in an aqueous solution. The solution may contain buffers or salts to promote annealing, and/or stabilization of the duplex strands.

It will be appreciated that a polynucleotide agent of the present invention may be provided per se, or as a nucleic acid construct comprising a nucleic acid sequence encoding the polynucleotide agent.

Typically, the nucleic acid construct comprises a promoter sequence which is functional in the host cell, as detailed herein below.

The polynucleotide sequences of the present invention, under the control of an operably linked promoter sequence, may further be flanked by additional sequences that advantageously affect its transcription and/or the stability of a resulting transcript. Such sequences are generally located upstream of the promoter and/or downstream of the 3' end of the expression construct.

The term "operably linked", as used in reference to a regulatory sequence and a structural nucleotide sequence, means that the regulatory sequence causes regulated expression of the linked structural nucleotide sequence. "Regulatory sequences" or "control elements" refer to nucleotide sequences located upstream, within, or downstream of a structural nucleotide sequence, and which influence the timing and level or amount of transcription, RNA processing or stability, or translation of the associated structural nucleotide sequence. Regulatory sequences may include promoters, translation leader sequences, introns, enhancers, stem-loop structures, repressor binding sequences, termination sequences, pausing sequences and polyadenylation recognition sequences.

It will be appreciated that the nucleic acid agents can be delivered to the Varroa mites in a great variety of ways.

According to one embodiment, the nucleic acid agents are delivered directly to the mites (e.g. by spraying an infested hive). The nucleic acid agents, or constructs encoding same may enter the mites bodies by diffusion. In this embodiment, the promoter of the nucleic acid construct is typically operational in mite cells.

It will be appreciated that since Varroa mites use their mouths to puncture the bee exoskeleton and feed on the bee's hemolymph, the present invention contemplates delivering the polynucleotide agents of the present invention to the bees, whereby they become presented in the bee's hemolymph thereby becoming available to the mite. Thus, according to another embodiment, the nucleic acid agents are delivered indirectly to the mites (e.g. via the bee). In this embodiment, the promoter of the nucleic acid construct is typically operational in bee cells.

According to one embodiment, the nucleic acid agents are delivered to the bees by spraying. The nucleic acid agents, or constructs encoding same may enter the bees bodies by diffusion.

According to another embodiment, the nucleic acid agents are delivered to the bees via its food. The present inventors consider that following ingestion of the nucleic acid agents, the agents will be presented in the bee's hemolymph, whereby it becomes available to the Varroa mite.

Thus the polynucleotides of the present invention may be synthesized *in vitro* and added to the food. For example double stranded RNA may be synthesized by adding two opposing promoters (e.g. T7 promoters; SEQ ID NOs: 48 and 49) to the ends of the gene segments, wherein SEQ ID NO: 48 is placed immediately 5' to the gene and SEQ ID NO: 49 is placed immediately 3' to the gene segment. The dsRNA may then be transcribed in vitro with the T7 RNA polymerase.

Exemplary sequences for synthesizing dsRNA according to embodiments of the present invention are provided in SEQ ID NOs: 50-90.

As detailed herein, bee feeding is common practice amongst bee-keepers, for providing both nutritional and other, for example, supplemental needs. Bees typically feed on honey and pollen, but have been known to ingest non-natural feeds as well. Bees can be fed various foodstuffs including, but not limited to Wheast (a dairy yeast grown on cottage cheese), soybean flour, yeast (e.g. brewer's yeast, torula yeast) and yeast products products-fed singly or in combination and soybean flour fed as a dry mix or moist cake inside the hive or as a dry mix in open feeders outside the hive. Also useful is sugar, or a sugar syrup. The addition of 10 to 12 percent pollen to a supplement fed to bees improves palatability. The addition of 25 to 30 percent pollen improves the quality and quantity of essential nutrients that are required by bees for vital activity.

Cane or beet sugar, isomerized corn syrup, and type-50 sugar syrup are satisfactory substitutes for honey in the natural diet of honey bees. The last two can be supplied only as a liquid to bees.

Liquid feed can be supplied to bees inside the hive by, for example, any of the following methods: friction-top pail, combs within the brood chamber, division board feeder and boardman feeder. Dry sugar may be fed by placing a pound or two on the inverted inner cover. A supply of water must be available to bees at all times. In one embodiment, pan or trays in which floating supports-such as wood chips, cork, or plastic sponge-are present are envisaged. Detailed descriptions of supplemental feeds for bees can be found in, for example, USDA publication by Standifer, et al 1977, entitled "Supplemental Feeding of Honey Bee Colonies" (USDA, Agriculture Information Bulletin No. 413).

It will be appreciated that Varro mites cause wound sites in the exoskeleton of bees. Such wound sites harbor bacterial infections, such as Melissococcus pluton, which causes European foulbrood. In addition, to their parasitic effects, Varroa mites are suspected of acting as vectors for a number of honey bee pathogens, including deformed wing virus (DWV), Kashmir bee virus (KBV), acute bee paralysis virus (ABPV) and black queen cell virus (BQCV), and may weaken the immune systems of their hosts, leaving them vulnerable to infections.

Thus, by killing the mites (or preventing reproduction thereof), the agents of the present invention may be used to prevent and/or treat bacterial infections such as Melissococcus pluton and viral infections caused by the above named viruses.

Since Varroa mite infestation and viral infections are thought to be responsible for colony collapse disorder (CCD), the present agents may also be used to prevent or reduce the susceptibility of a bee colony to CCD.

It will be appreciated that in addition to feeding of oligonucleotides and/or polynucleotides for reduction of the bee pathogen infection and infestation, enforcement of proper sanitation (for example, refraining from reuse of infested hives) can augment the effectiveness of treatment and prevention of infections.

It is expected that during the life of a patent maturing from this application many relevant methods for downregulating expression of gene products will be developed and the scope of the term "downregulating expression of a gene product of a Varroa destructor mite" is intended to include all such new technologies *a priori.*

As used herein the term "about" refers to ± 10 %.

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

As used herein the term "method" refers to manners, means, techniques and procedures for accomplishing a given task including those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### EXAMPLE 1

### Feeding Varroa-specific dsRNA prevents Varroa mite infestation

In order to determine the effectiveness of ingested dsRNA on Varroa mite infestation, honeybees are provided with Varroa mite-specific and control dsRNA in the feed for 7 days before, and 2 days following contact with the Varroa mite, as illustrated in Figure 1. Numbers of dead Varroa per experimental hive are counted, and sample live and dead Varroa are collected for molecular analysis.

### MATERIALS AND METHODS

***Establishment of mini-hive colonies:*** Young, approximately 2-month-old queens, together with approximately 200 worker bees are collected from hives in a local apiary. The bees are transferred into mini-hives fitted with one mini comb that was previously built by a regular hive. All of the mini-hives are closed and placed in a temperature-controlled room (30°C).

***dsRNA preparation:*** Varroa mite sequences are cloned into a plasmid between two opposing T7 promoters. Following propagation of plasmid DNA, the viral fragments, including the T7 promoters, are excised and gel-purified. These serve as templates for T7-directed *in-vitro* transcription (MEGAscript™, Ambion, Austin TX). The reaction product is submitted to DNase digestion followed by phenol extraction and ethanol precipitation. The final preparation is dissolved in nuclease-free water.

***dsRNA feeding in minihives**:* 5 gr. pollen supplement patties are placed on top of each comb and 10 ml of 50 % sucrose solution is introduced into the hive in a sterile Petri dish nightly. The feeding is continued for 9 days and subsequently only hives in which queens had begun to lay eggs are included in the trial.

Following establishment of active hives (queens laying eggs), some of the mini-hives are supplemented with Varroa mite-specific (apoptosis inhibitor (IAP) gene (SEQ ID NO: 27) or non-specific control (e.g. GFP SEQ ID NO: 91) dsRNA, which is added to the 10 ml 50 % sugar solution given to the hives, adjusted to approximately 1 microgram dsRNA per feed per bee, assuming all bees consume approximately the same amount of sucrose solution. dsRNA feeding is continued for six days.

***Varroa mite infestation in minihives:*** 7 days after feeding in active hives, some of the colonies are placed in contact with a population of Varroa mites. Thereafter, dsRNA treatment is continued for a further 2 days. Samples of live and dead bees (larvae and adults) are collected daily from each mini-hive post introduction of the Varroa mite population for 32 consecutive days. Every bee collected is frozen in liquid nitrogen and preserved at -70 °C pending molecular analysis. Vitality of the colonies are monitored by opening the hives (without smoke), withdrawing the mini-comb and photographing the mini-comb from both sides. The hive-combs are photographed daily, and the numbers of remaining live bees are monitored. The photographs are downloaded onto a computer and the total number of bees is counted for every mini-hive.

To test dsRNA toxicity, another group of hives are provided with Varroa mite-specific dsRNA, but is not placed in contact with the Varroa mite population. Two sets of hives serve as additional controls: hives that are not treated with dsRNA and are not inoculated with Varroa mites, and hives that were not treated with dsRNA, but were inoculated with Varroa mites.

### RT-PCR analysis:

***Extraction of Nucleic Acids:*** Total RNA is extracted from the preserved bees using the TRIREAGENT method (Sigma, St. Louis MO, USA). Briefly, RNA is extracted by precipitation and separation by centrifugation, then resuspended in RNAsecure solution.

***Real-Time RT-PCR:*** Measured amounts of RNA (100 ng for viral expression analyses and 100 pg for 18S rRNA internal controls) are subjected to one-step RT-PCR using the SYBR Green PCR master mix with Taqman reverse transcriptase (Applied Biosystems, Foster City, CA). Real-time RT-PCR is conducted in GeneAmp PCR System 5700 (Applied Biosystems). Reactions performed without reverse transcriptase or without template should not result in any product.

***Northern***-***Blot Analysis:*** Total RNA is extracted from treated and control bees. Formaldehyde is added to the RNA to 1.8 % and warmed to 65 °C. The RNA, 15 *µ*g per lane is electrophoresed on a 1.2 % agarose gel at 70 V, 4 °C with stirring. The previously described amplified Varroa mite-RNA product is digoxigenin labeled and serves as a probe for hybridization. Detection is performed with the DIG luminescent detection kit (Roche Diagnostics GmbH, Mannheim, Germany). RNA sizes are estimated by comparison to electrophoresed RNA Molecular Weight Markers I (Roche). Hybridization is carried out at high stringency (0.1x SSC; 65°C).

*T****he fate of ingested Varroa mite*-*specific dsRNA in honeybees:*** In order to better understand the mechanism(s) of action by which dsRNA-Varroa mite protects the bees against Varroa mite infestation and its consequences, total RNA is extracted from dsRNA-Varroa mite treated, and non-treated control bees, submitted to digestion by a panel of nucleases, and separated on PAGE

### RESULTS

The presence of dsRNA in the adult bee body in the bee larvae (fed by adult bees), in the bee pupa was determined by slot-blot hybridization with a probe for GFP. The processing of the dsRNA to siRNA was determined by Northern blots detecting small RNAs (Figures 2A-E).

Varroa individuals were placed on adult bees that had been fed for 7 days with dsRNA-GFP and on control (unfed) bees. RNA was extracted from Varroa at the indicated times (Figure 1) and subjected to RT-PCR with GFP primers. The results are illustrated in Figure 3.

Bees were fed with a segment of dsRNA for apoptosis inhibitor (IAP) gene (SEQ ID NO: 27). Varroa collected from that hive were analyzed by RT-PCR for the expression of the IAP gene (Figure 4).

### EXAMPLE 2

### MATERIALS AND METHODS

Hives were fed by two different mixtures of dsRNAs corresponding to Varroa gene segments. All dsRNA were corresponding to gene segments that are not homologous to bee or human sequences (not carrying stretches of homologous sequences longer than 19 bases). Mixture I (Minimum treatment) contained SEQ ID NOs: 1, 13, 27, 30 and 39. Mixture II (Maximum treatment) contained SEQ ID NOs: 1, 4, 7, 10, 13, 16, 19, 22, 25, 27, 30, 33, 36 and 39. Thirty Varroa individuals were placed in each hive and two months later Varroa and bees were counted in each hive. Each treatment was repeated 3 times.

### RESULTS

No visible damage to the strength of the hive was noticed among the various hives. Figure 5 demonstrates the reduction of Varroa population following treatment with dsRNAs of Varroa's gene sequences.

### EXAMPLE 3

### Large-scale field trials of Varroa-specific dsRNA for prevention of Varroa mite-associated disease of honeybees

In order to determine the effectiveness of ingested Varroa mite dsRNA on Varroa mite infestation under actual field conditions, and to assess effects on important parameters of colony health, bees in sample full size hives are provided with Varroa mite-specific dsRNA in the feed for 5 days before, and 4 days following infestation with Varroa mite.

### MATERIALS AND METHODS

### Insect material:

Pools of five bees from the following treatments; Remote control, Varroa mite-dsRNA only, Varroa mite only and Varroa mite-specific dsRNA+ Varroa mite at each time point day 0-(day of virus application), day 7 and end point (day 42). The test was repeated several times.

### RNA extraction:

RNA is extracted using Tri-Reagent (Sigma,USA) according to protocol provided by the manufacturer. All samples are treated with DNaseI and resuspended with loading buffer (90 % Formamide, 0.05 Bromophenol Blue, 0.05 % Xylene cyanol) prior to loading on gel.

### Gel electrophoresis and Blot:

10 ug of freshly prepared RNA is measured using the nanodrop spectrophotometer and loaded on 12 % Acrylamide gel (1:19 acrylamide:Bis acrylamide ratio) in danturation environment (gel contains 7M Urea). After electrophoresis samples are transferred to positively charged nylon membrane (Roch,USA) using electrobloting method.

### Hybridization and signal detection:

Membrane is hybridized with freshly prepared DNA probe of Varroa mite segment, taken from a region that does not correspond to the dsRNA of the Varroa mite-specific dsRNA itself. This is made using DIG PCR probe preparation Kit (Roch,USA) o/n 42°C in DIG easyhyb solution (Roch, USA) according to manufacturer protocol. The membrane is washed twice with 2 X SSC/0.1 % SDS, than washed for stringency with 0.1 X SSC/0.1 % SDS in 65 °C. Membranes are further washed using DIG Wash and Block Kit (Roch, USA) according to manufacturer protocol. Detection is preformed using CSPD-star substrate (Roch, USA). Positive control is 21nt DNA primers corresponding to the hybridized sequence.

Signal is detected using membrane exposure for 2-12 hours in chemiluminator manufactured by Kodak.

Basic parameters of bee colony health (numbers of capped brood, numbers of bees in the hive, returning foragers and honey production) are assessed in hives fed Varroa mite-dsRNA and control hives, in the absence of Varroa mite infestation.

## Claims

1. A bee-ingestible composition comprising at least one nucleic acid agent comprising a nucleic acid sequence which downregulates expression of a Varroa destructor mite-specific mRNA for use in preventing or treating a Varroa destructor mite infestation of a bee hive, wherein said mRNA encodes a polypeptide selected from the group consisting of ATPase subunit A, RNA polymerase I, RNA polymerase III, Inhibitor of apoptosis (IAP), FAS apoptotic inhibitor and α-Tubulin, wherein said agent is a double stranded RNA and wherein the double stranded RNA is at least 100 bases long and does not comprise any sequence longer than 19 bases entirely homologous to any bee-genome sequence or human-genome RNA sequence.

2. The bee-ingestible composition of claim 1, wherein said double stranded RNA targets a gene segment consisting of a sequence of SEQ ID NO: 1, 4, 6, 7 (ATPase subunit A), 10 (RNA polymerase I), 13, 16, 18, 19 (RNA polymerase III), 22, 23, 25, 27 (IAP), 30, 31 (FAS apoptotic inhibitor), 33, 36, 38, 39, or 41 (α-Tubilin).

3. The bee-ingestible composition of claim 1, wherein said bee-ingestible composition comprises a mixture containing dsRNAs defined by SEQ ID NOs: 1, 13, 27, 30, and 39.

4. The bee-ingestible composition of claim 1, wherein said bee-ingestible composition comprises a mixture containing dsRNAs defined by SEQ ID NOs: 1, 4, 7, 10, 13, 16, 19, 22, 25, 27, 30, 33, 36, and 39.

5. The bee-ingestible composition of any one of claims 1-4, wherein said composition is in a solid form.

6. The bee-ingestible composition of any one of claims 1-4, wherein said composition is in a liquid form.

7. The bee-ingestible composition of any one of claims 1-6, wherein said composition comprises protein.

8. The bee-ingestible composition of claim 7, wherein said protein is in the form of pollen and/or soy patties.

9. The bee-ingestible composition of claim 6, wherein said composition is in liquid form and further comprises a carbohydrate or sugar supplement.

## Patentansprüche

1. Von Bienen konsumierbare Zusammensetzung, die wenigstens ein Nucleinsäuremittel umfasst, das eine Nucleinsäuresequenz umfasst, die die Expression einer *Varroa-destructor-*Milben-spezifischen mRNA herunterreguliert, zur Verwendung bei der Prävention oder Behandlung eines Befalls eines Bienenstocks mit *Varroa-destructor-*Milben, wobei die mRNA ein Polypeptid codiert, das aus der Gruppe ausgewählt ist, die aus ATPase-Untereinheit A, RNA-Polymerase I, RNA-Polymerase III, einem Apoptoseinhibitor (IAP), einem FAS-Apoptoseinhibitor und α-Tubulin besteht, wobei das Mittel eine doppelsträngige RNA ist und wobei die doppelsträngige RNA wenigstens 100 Basen lang ist und keine Sequenz mit einer Länge von mehr als 19 Basen umfasst, die irgendeiner Bienengenomsequenz oder RNA-Sequenz des humanen Genoms vollständig homolog ist.

2. Von Bienen konsumierbare Zusammensetzung gemäß Anspruch 1, wobei die doppelsträngige RNA gegen ein Gensegment gerichtet ist, das aus einer Sequenz von SEQ ID Nr. 1, 4, 6, 7 (ATPase-Untereinheit A), 10 (RNA-Polymerase I), 13, 16, 18, 19 (RNA-Polymerase III), 22, 23, 25, 27 (IAP), 30, 31 (FAS-Apoptoseinhibitor), 33, 36, 38, 39 oder 41 (α-Tubulin) besteht.

3. Von Bienen konsumierbare Zusammensetzung gemäß Anspruch 1, wobei die von Bienen konsumierbare Zusammensetzung ein Gemisch umfasst, das dsRNAs enthält, die durch SEQ ID Nr. 1, 13, 27, 30 und 39 definiert sind.

4. Von Bienen konsumierbare Zusammensetzung gemäß Anspruch 1, wobei die von Bienen konsumierbare Zusammensetzung ein Gemisch umfasst, das dsRNAs enthält, die durch SEQ ID Nr. 1, 4, 7, 10, 13, 16, 19, 22, 25, 27, 30, 33, 36 und 39 definiert sind.

5. Von Bienen konsumierbare Zusammensetzung gemäß einem der Ansprüche 1 bis 4, wobei die Zusammensetzung in fester Form vorliegt.

6. Von Bienen konsumierbare Zusammensetzung gemäß einem der Ansprüche 1 bis 4, wobei die Zusammensetzung in flüssiger Form vorliegt.

7. Von Bienen konsumierbare Zusammensetzung gemäß einem der Ansprüche 1 bis 6, wobei die Zusammensetzung Protein umfasst.

8. Von Bienen konsumierbare Zusammensetzung gemäß Anspruch 7, wobei das Protein in Form von Pollen und/oder Sojaplätzchen vorliegt.

9. Von Bienen konsumierbare Zusammensetzung gemäß Anspruch 6, wobei die Zusammensetzung in flüssiger Form vorliegt und weiterhin eine Kohlenhydrat- oder Zuckerergänzung umfasst.

## Revendications

1. Composition consommable par les abeilles comprenant au moins un agent d'acide nucléique comprenant une séquence d'acide nucléique qui régule négativement l'expression d'un ARNm spécifique de l'acarien Varroa destructor pour une utilisation dans la prévention ou le traitement d'une infestation par l'acarien Varroa destructor d'une ruche d'abeilles, dans laquelle ledit ARNm code pour un polypeptide choisi dans le groupe constitué de la sous-unité A de l'ATPase, l'ARN polymérase I, l'ARN polymérase III, l'inhibiteur de l'apoptose (IAP), l'inhibiteur apoptosique FAS et l'α-tubuline, dans laquelle ledit agent est un ARN double brin et dans lequel l'ARN double brin a une longueur d'au moins 100 bases et ne comprend aucune séquence d'une longueur supérieure à 19 bases entièrement homogène à une séquence quelconque du génome de l'abeille ou à une séquence quelconque d'ARN du génome humain.

2. Composition consommable par les abeilles selon la revendication 1, dans laquelle ledit ARN double brin cible un segment de gène constitué d'une séquence de SEQ ID NO : 1, 4, 6, 7 (sous-unité A de l'ATPase), 10 (ARN polymérase I), 13, 16, 18, 19 (ARN polymérase III), 22, 23, 25, 27 (IAP), 30, 31 (inhibiteur apoptosique FAS), 33, 36, 38, 39, ou 41 (α-tubuline).

3. Composition consommable par les abeilles selon la revendication 1, dans laquelle ladite composition consommable par les abeilles comprend un mélange contenant des ARNdb définis par SEQ ID NO : 1, 13, 27, 30, et 39.

4. Composition consommable par les abeilles selon la revendication 1, dans laquelle ladite composition consommable par les abeilles comprend un mélange contenant des ARNdb définis par SEQ ID NO : 1, 4, 7, 10, 13, 16, 19, 22, 25, 27, 30, 33, 36, et 39.

5. Composition consommable par les abeilles selon l'une quelconque des revendications 1 à 4, dans laquelle ladite composition est sous une forme solide.

6. Composition consommable par les abeilles selon l'une quelconque des revendications 1 à 4, dans laquelle ladite composition est sous une forme liquide.

7. Composition consommable par les abeilles selon l'une quelconque des revendications 1 à 6, dans laquelle ladite composition comprend une protéine.

8. Composition consommable par les abeilles selon la revendication 7, dans laquelle ladite protéine est sous la forme de galettes de pollen et/ou de soja.

9. Composition consommable par les abeilles selon la revendication 6, dans laquelle ladite composition est sous forme liquide et comprend en outre un complément de glucide ou de sucre.
